# EUROPEAN PATENT SPECIFICATION

(11) **EP 2 613 721 B1**
(45) Date of publication and mention of the grant of the patent: **23.08.2017**
(21) Application number: 11840437.5
(22) Date of filing: 12.09.2011
(51) Int. Cl.: A61B 17/72, A61B 17/86, A61B 17/88

(54) **PROXIMAL INTERPHALANGEAL FUSION DEVICE**
VORRICHTUNG ZUR VERKNÜPFUNG BENACHBARTER KNOCHENGELENKE
DISPOSITIF DE FUSION INTER-PHALANGIENNE PROXIMALE

(30) Priority: 16.03.2011 US 201113049363; 10.09.2010 US 381732 P
(43) Date of publication of application: 17.07.2013
(73) Proprietor: Competitive Global Medical, LLC, Tucson, AZ 85706 (US)
(72) Inventor: CHAMPAGNE, Lloyd, Paradise Valley, AZ 85253 (US); KING, Bruce, Tucson, AZ 85750 (US); CONTENTO, Omar, Hillsboro, OR 97123 (US); LANHAM, Mike, Tucson, AZ 85730 (US); MARTIN, Donald, J., Tucson, AZ 85715 (US)
(74) Representative: Bittner, Bernhard
(86) International application number: PCT/US2011/051256
(87) International publication number: WO 2012/064401

(56) References cited:
- WO-A1-97/33537
- WO-A1-2010/096724
- US-A- 5 417 692
- US-A- 5 827 285
- US-A- 5 984 970
- US-A1- 2002 198 527
- US-A1- 2004 172 031
- US-A1- 2005 038 438
- US-A1- 2006 271 054
- US-A1- 2010 036 439
- US-B1- 7 041 106

## Description

Hammertoe is the most common deformity of the lesser toes and is characterized by an abnormal flexion posture of the proximal interphalangeal (PIP) joint of at least one of the lesser toes, which gives the affected toe a bent, claw-like appearance. The factors contributing to hammer toe are difficult to determine. Women are more commonly affected, and the incidence of the deformity increases with age. Though diabetes, connective tissue disease, and trauma are identified as predisposing factors, the long-term use of poorly fitting shoes, especially those with a tight toe box, is generally thought to be a primary cause of the condition. The resulting crowding and overlapping of the toes while wearing the shoes may lead to either flexible deformities, which can be passively corrected to a neutral position, or fixed deformities, which cannot be passively corrected.

In addition to having cosmetic complaints about the hammer toe, patients with the deformity also commonly experience pain on the dorsum, or top side, of the PIP joint, where a corn typically develops from the pressure that the poorly fitting shoe exerts on the toe. Painful calluses may also develop at the distal end of the affected toe or beneath the metatarsal head. Other underlying conditions, such as diabetes, may cause ulceration and possibly infection to develop at these areas of pressure, further complicating treatment and endangering the toe or foot.

Several different treatment options exist for hammer toe, with varying degrees of success. For flexible deformities, a physician may prescribe more conservative passive treatment, such as stretching the toe, taping the toe, soft shoe inserts, or shoes with wider toe boxes. However, these treatments are generally disappointing, with recurrence of the hammer toe likely once the treatment stops. Most patients with symptomatic hammer toe, as well as those with a fixed deformity, will require surgical treatment. Soft-tissue procedures alone, such as flexor-to-extensor tendon transfer, may not lead to permanent correction and are not appropriate for all patients. Thus, bone and joint procedures are more commonly used to surgically correct hammer toe.

Resection and arthrodesis of the PIP joint are among the most common surgical procedures for treating very rigid deformities or when additional joint stability is needed. Arthrodesis alleviates pain by promoting fusion of adjacent bones at the joint. Fusion can be accomplished using K-wires, orthopedic screws, or plates. However, a straight fusion usually looks unnatural, as a degree of flexion is normal at the PIP joint. Better options for PIP fusion are needed to meet patients' needs.

Traditionally, K-wires have been the most common means for completing bone fusion procedures. However, K-wires have several drawbacks. Bone fusion by K-wire involves placing the toe in the correct anatomical position and then driving the K-wire through both bones and the joint to hold them in place. Part of the K-wire protrudes out of the tip of the toe to facilitate removal of the K-wire several weeks later. This aspect of the procedure greatly increases the risk of infection, in addition to requiring multiple dressing changes and limiting ambulation. Other complications that commonly arise with the use of K-wires include K-wire migration, loss of fixation, misalignment, nonunion, swelling, and inflammation.

Recently, hammer toe implant devices have become more popular for fusion procedures. These devices may be composed of various biocompatible metal or thermoplastic materials and remain in the toe, providing increased stability. However, common complications that are associated with these devices include bony regrowth, prolonged edema, limited range of motion, poor toe purchase, and the need to remove the implant in certain situations. Furthermore, these devices typically have a linear shape, leaving the bones fused in an unnatural, straight conformation. One example for such an implant device is disclosed in US 2005/0038438 A1. This document describes a simple, safe implantable bone screw, and a method for distracting two bone segments. The bone screw includes a shank having a threaded proximal portion and a distal portion with a major diameter that is less than a minor diameter of the threaded proximal portion. A transitional region of decreasing diameter can be disposed between the threaded proximal portion and the distal portion, and a driver receiving element is preferably disposed on a proximal end of the bone screw. In use, the proximal and distal portions of the bone screw are adapted to engage two segments of bone, and to create a distraction force between the two segments of bone.

Furthermore WO 2010 096 724 A1 discloses a bone joining device suitable for joining a first bone piece to a second bone piece. This device comprises a first component and a second component, wherein the first component comprises an elongated stem portion comprising a first end and a first top opposite the first end. This elongated stem portion can be inserted from the first end longitudinally into a surface of the first bone piece. The second component comprises a further elongated stem portion comprising a second end and a second top. This further elongated stem portion can be inserted from the second end longitudinally into a surface of the second bone piece. A connector which can be coupled and locked with the first component extends from the second top.

However, there still exists a need for fusion devices that avoid the above-mentioned complications, while providing the patient with a natural degree of flexion in the corrected toe.

Thus, a need for fusion devices exists which provide the patient with a natural degree of flexion in the bone. This problem is solved by the subject matter of the independent claims 1 and 8. Embodiments of the invention are directed to an improved implantable bone fusion apparatus for proximal interphalangeal ("PIP") fusion. Embodiments of the invention presented herein may be used in flexed or jointed bone fusion applications. In a flexible bone fusion application, an embodiment of the invention is directed to a bone fusion apparatus comprising an anchor portion having an elongated body with screw threads on at least a portion of its exterior, the anchor having a leading tip and an end; a compressor portion having an elongated body with screw threads on at least a portion of its exterior; and a coupler having a first end adapted to rotatably engage the anchor in a first plane, the coupler also having a second end adapted to rotatably engage the compressor through axial rotation.

Driving tools including a delivery tool and a compressor tool are provided for installing and then compressing the bones together, respectively. One embodiment is a two-part tool for driving or compressing a flexible bone fusion apparatus having a coupler portion, an anchor portion and a compressor portion, comprising a shaft portion and a separate handle portion, the shaft portion comprising a delivery shaft and two adjoining loading shafts at each end of the delivery shaft, the loading shafts adapted to couple with the handle portion and at least one driven or compressed component, each loading shaft having a driving pattern on its external surface that is complementary to a driving pattern on an internal surface of a lumen in one end of the driven or compressed component; and the handle portion having at one end a lumen adapted to receive the loading shaft of the shaft portion, the lumen having a driving pattern on its internal surface that is complementary to the driving pattern on the loading shaft. The invention is directed to a flexible bone fusion apparatus comprising:
(a) an anchor portion having an elongated body with screw threads on at least a portion of its exterior, the anchor having a leading tip and an end;
(b) a compressor portion having an elongated body with screw threads on at least a portion of its exterior; and
(c) a coupler having a first end adapted to rotatably engage the anchor portion in a first plane, the coupler also having a second end adapted to rotatably engage the compressor through axial rotation,
wherein the coupler and compressor portion are joined by a snap-fit interface comprising at least one annular ridge in either the compressor portion or coupler but not both, and at least one mating annular groove in the other of either the coupler or compressor portion but not both, said at least one groove being suitable to accommodate said at least one annular ridge wherein the diameter at the base of the at least one mating groove is the same as the external diameter of the at least one ridge such that the snap fit interface is adapted to allow relative axial rotation of the compressor portion and coupler.

Embodiments of the invention may also be directed to the flexible bone fusion apparatus wherein the coupler has an anchor end and a compressor end, the anchor and coupler sharing an interface at which they may rotate in a first plane, the coupler and compressor sharing a second interface at which they rotate axially.

Embodiments of the invention may also be directed to the flexible bone fusion apparatus wherein the compressor portion further comprises a lumen running the entire length of the compressor.

Embodiments of the invention may also be directed to the flexible bone fusion apparatus wherein when assembled the anchor and coupler share separate portions of an attachment assembly comprising an interlocking anchor attachment portion and a compressor attachment portion.

Embodiments of the invention may also be directed to the flexible bone fusion apparatus wherein the anchor attachment portion comprises at least one locking wedge, at least one semi-circular conical face and at least one semi-circular annular groove.

Embodiments of the invention may also be directed to the flexible bone fusion apparatus wherein the coupler attachment portion comprises at least one locking wedge, at least one semi-circular conical face and at least one semi-circular annular groove.

Embodiments of the invention may also be directed to the flexible bone fusion apparatus wherein the compressor lumen is adapted to engage a delivery tool through a driving pattern.

Embodiments of the invention may also be directed to the flexible bone fusion apparatus wherein the compressor lumen driving pattern comprises an octagonal cross-section having eight ridges.

Embodiments of the invention may also be directed to the flexible bone fusion apparatus wherein the compressor comprises at least one annular groove located in its lumen and the coupler comprises at least one annular ridge located on its external diameter.

Embodiments of the invention may also be directed to the flexible bone fusion apparatus wherein the snap-fit interface has at least one expansion slot cut through either the compressor body or coupler body or both.

Embodiments of the invention may also be directed to the flexible bone fusion apparatus wherein the screw threads of the anchor and compressor are synchronized to provide a continuous thread pitch from anchor to compressor.

Embodiments of the invention may also be directed to the flexible bone fusion apparatus wherein the first plane rotation direction varies from about 0° to about 30° when fixed in final position.

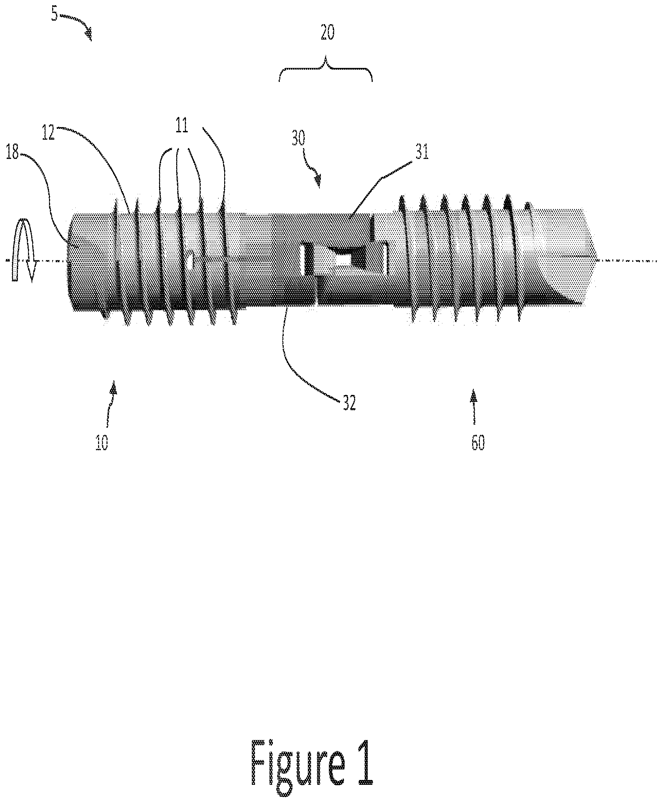

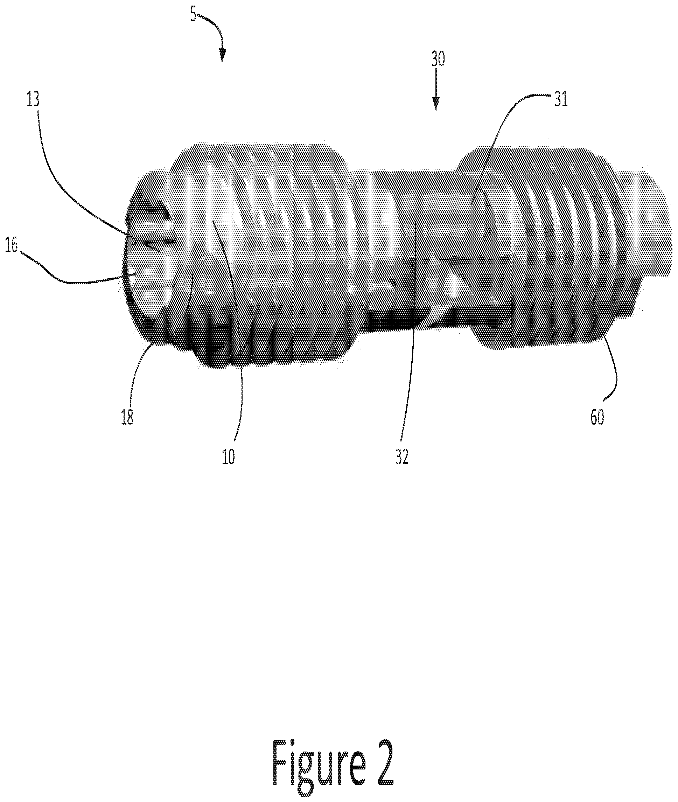

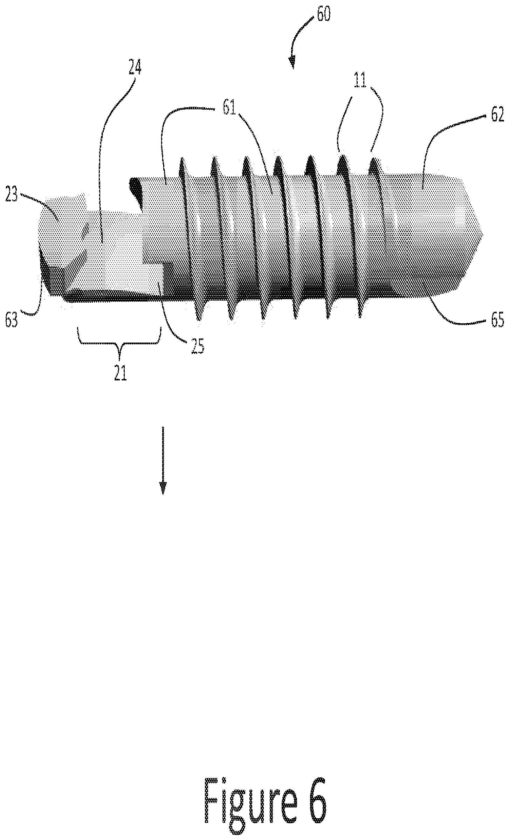

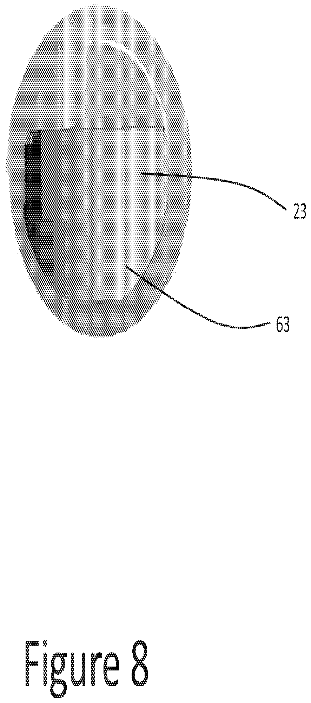

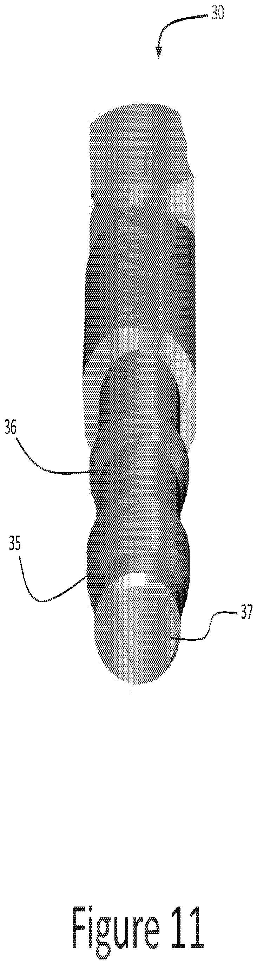

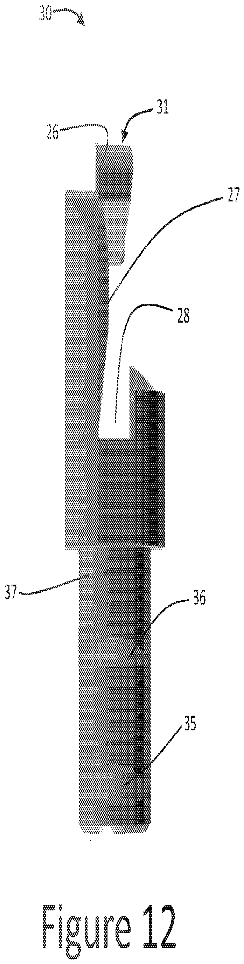

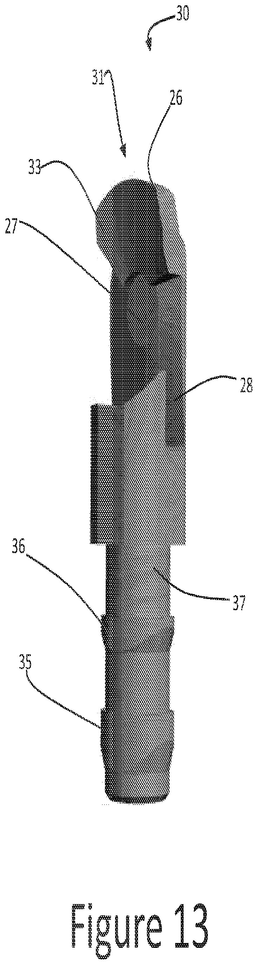

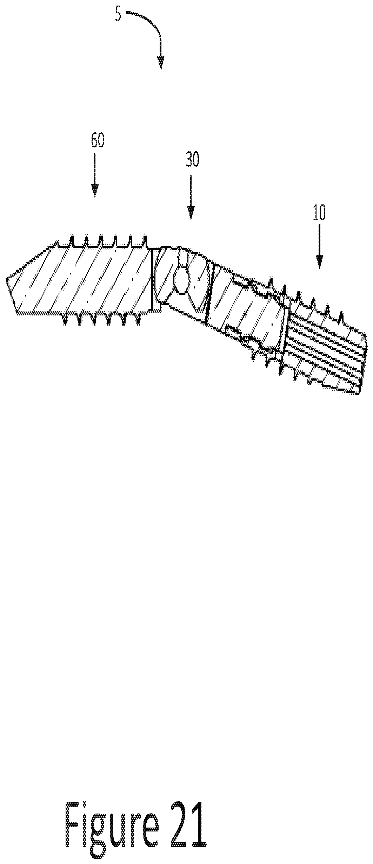

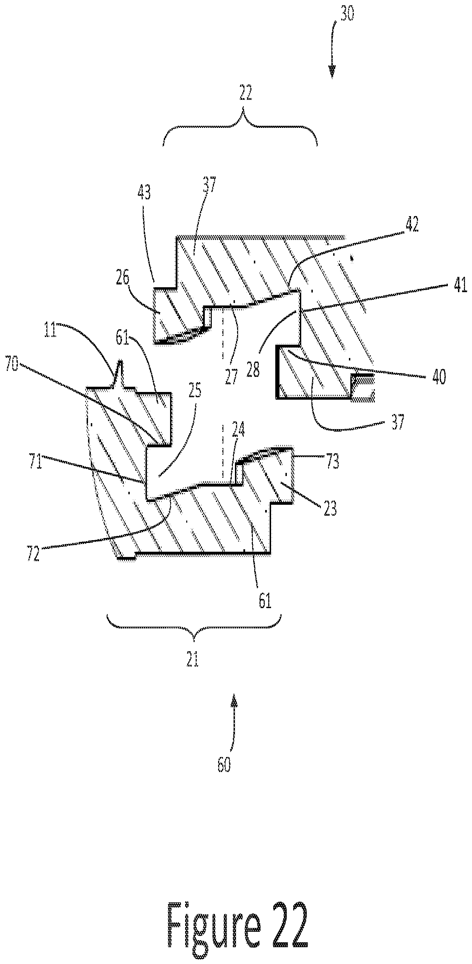

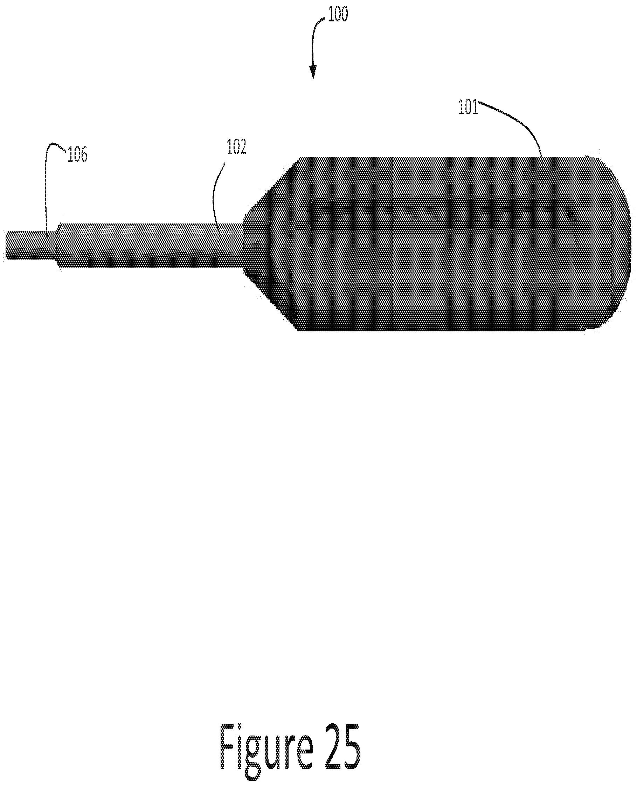

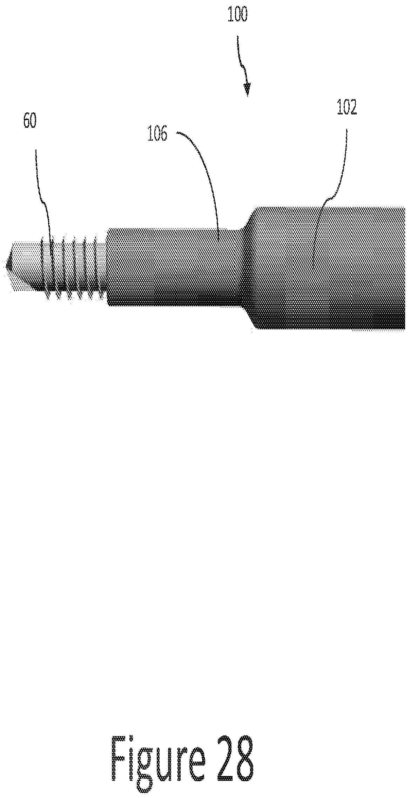

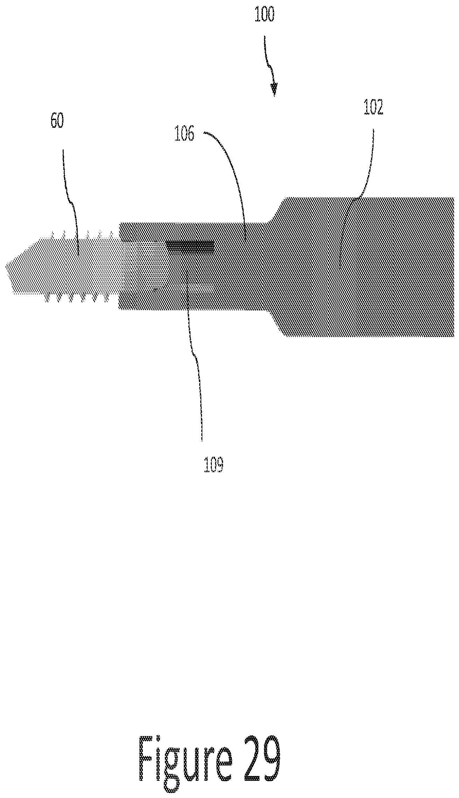

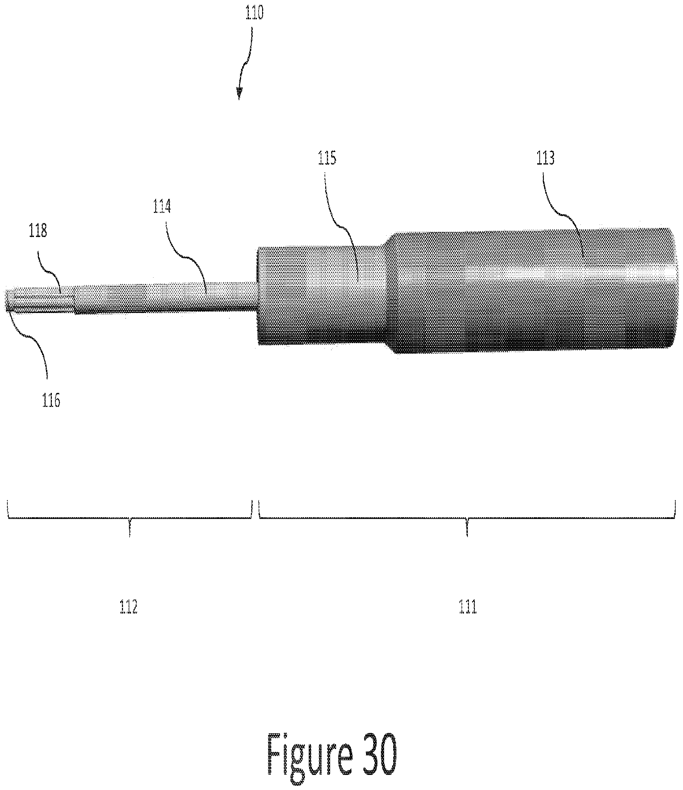

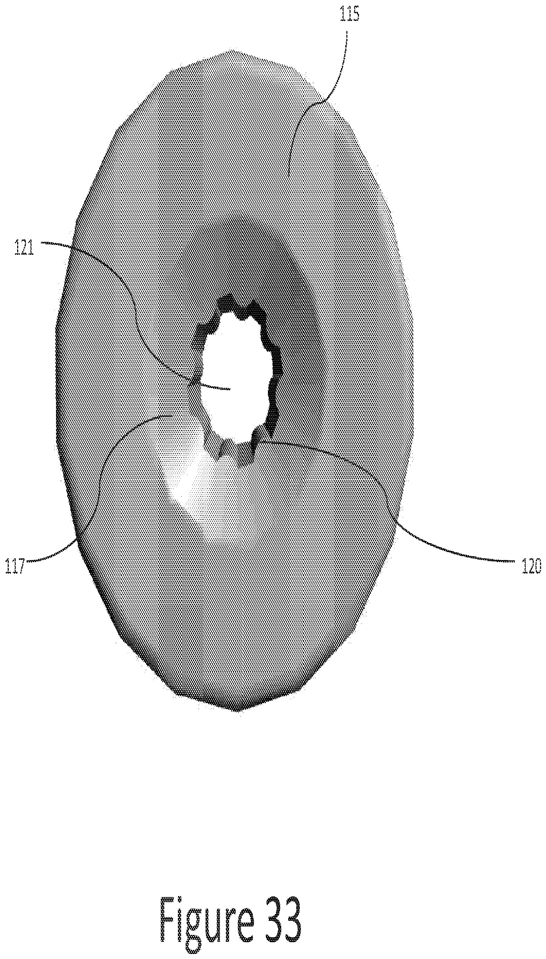

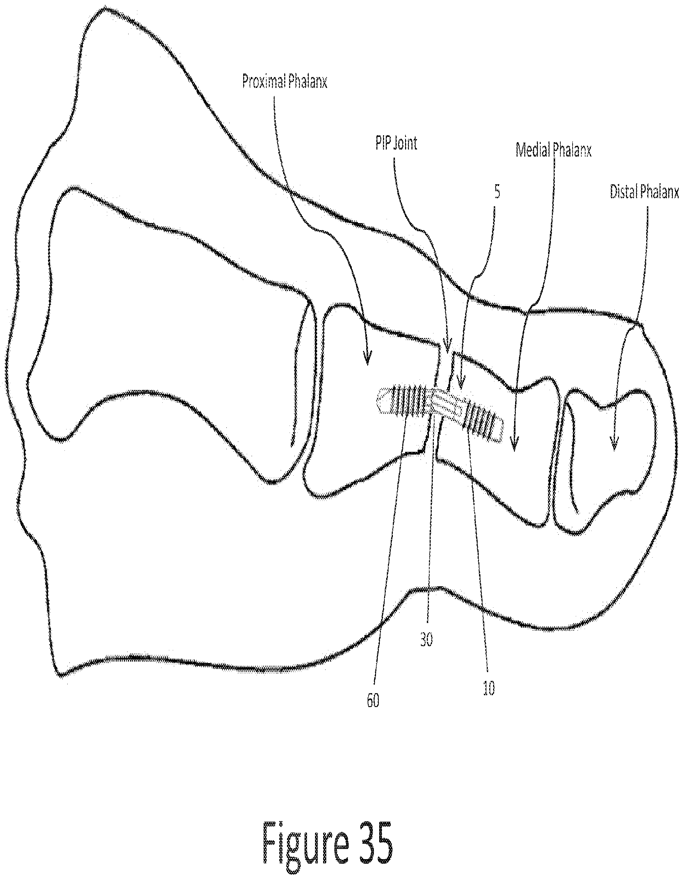

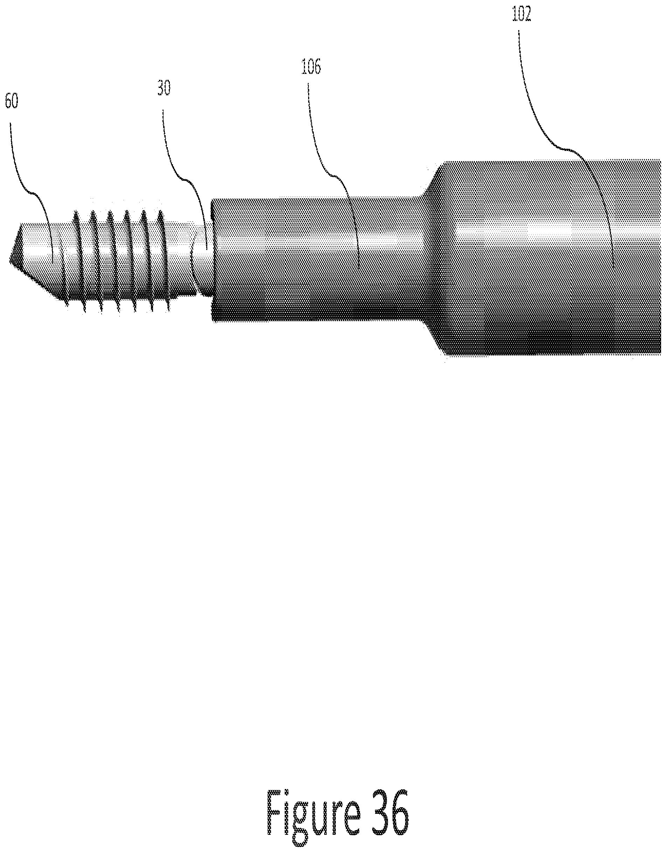

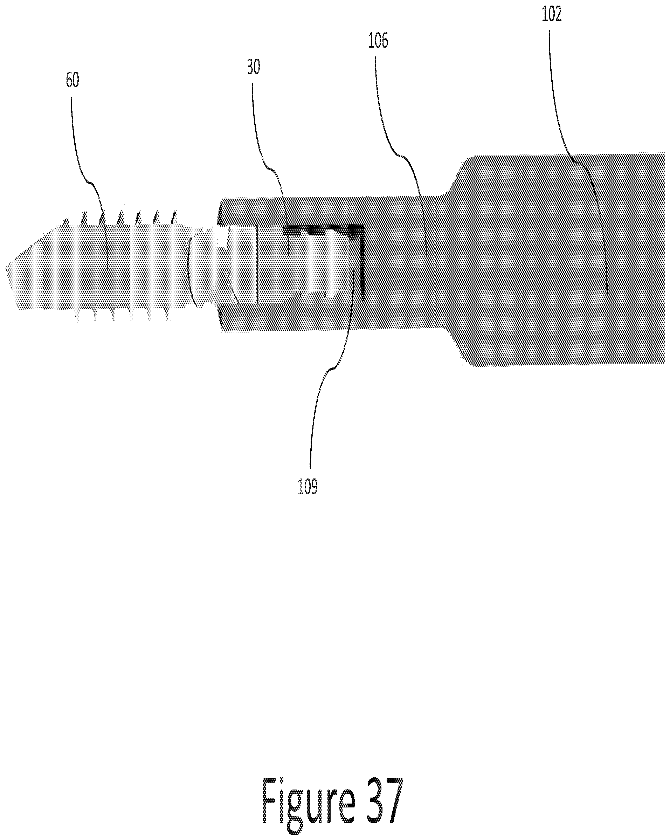

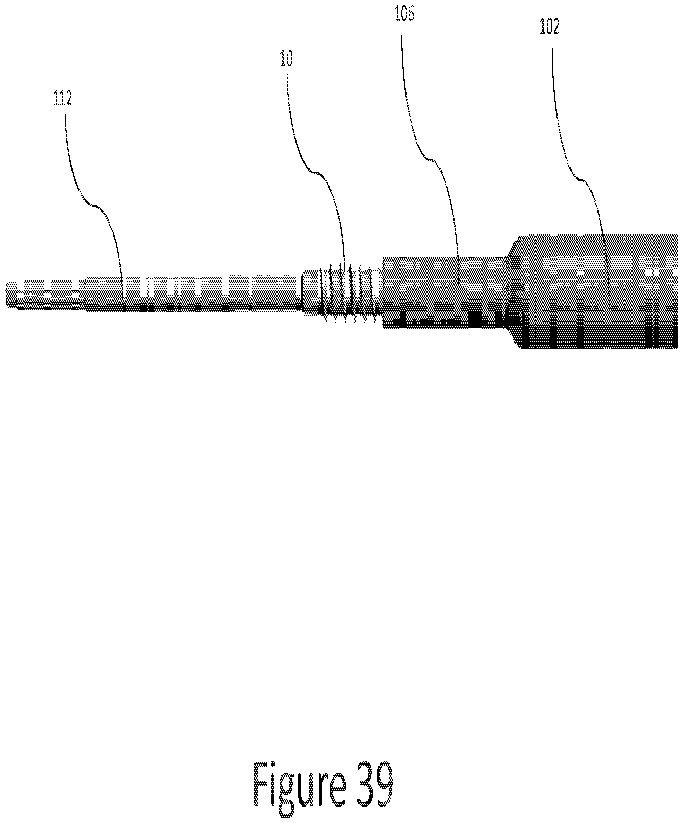

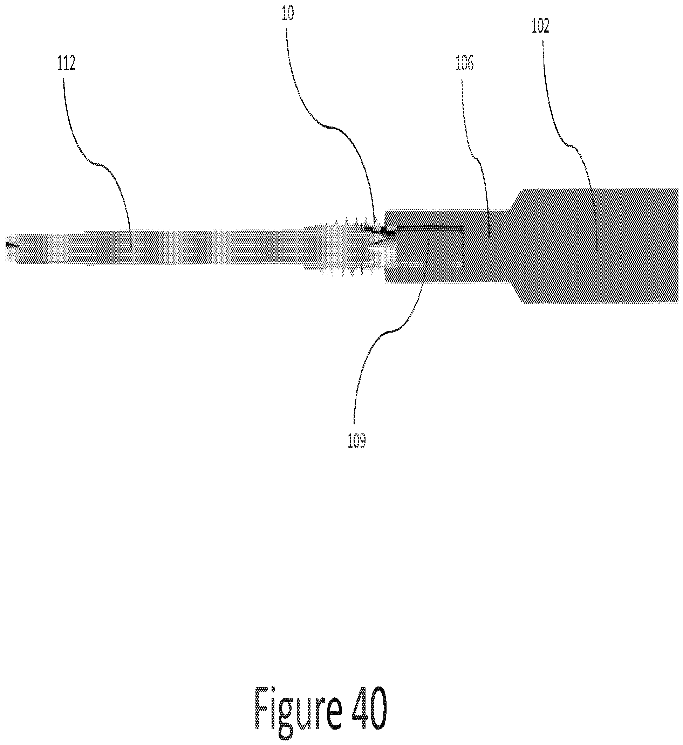

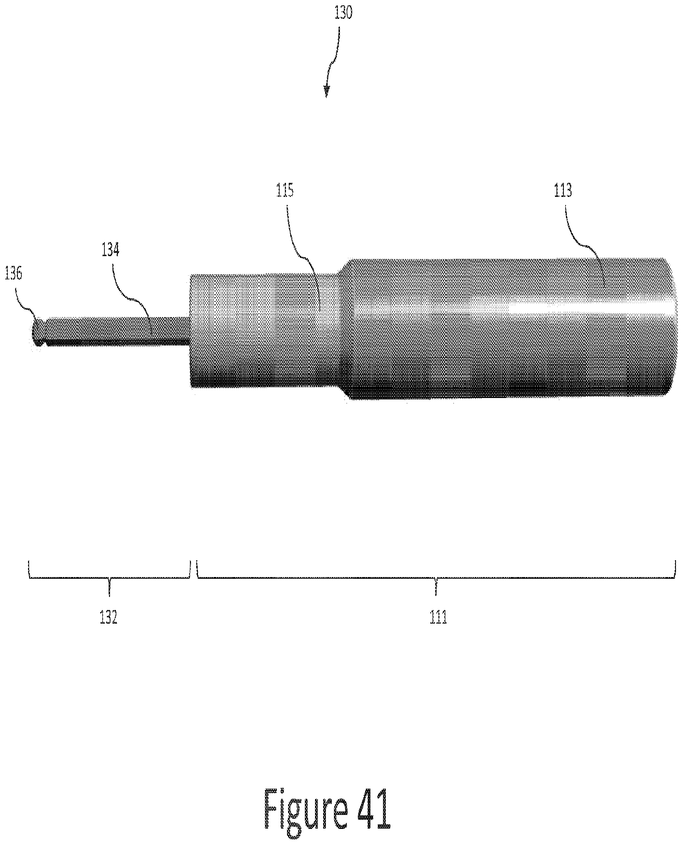

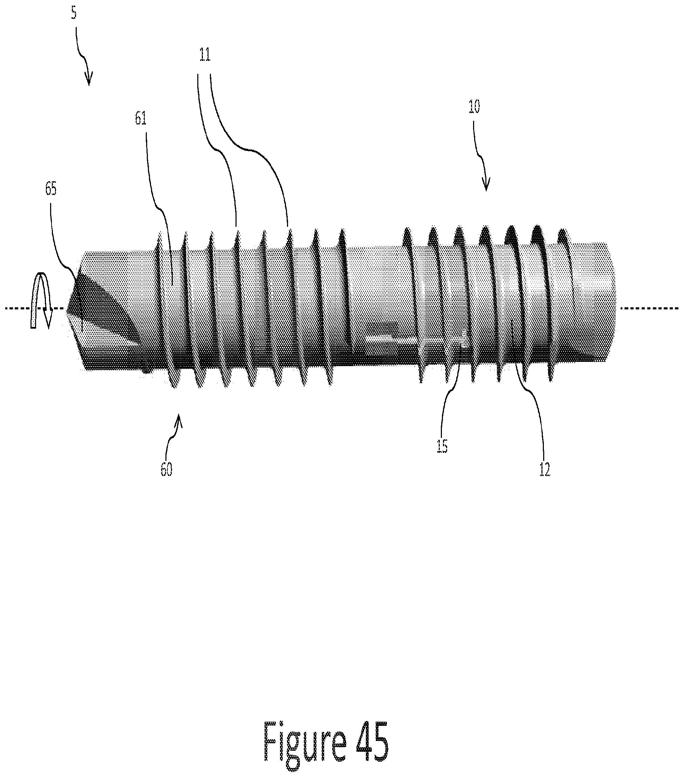

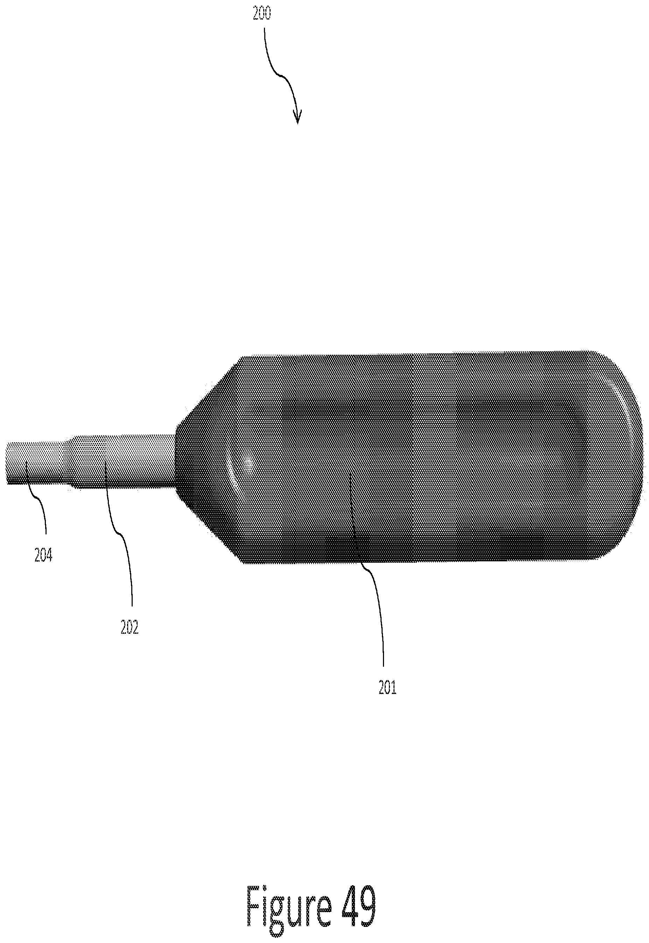

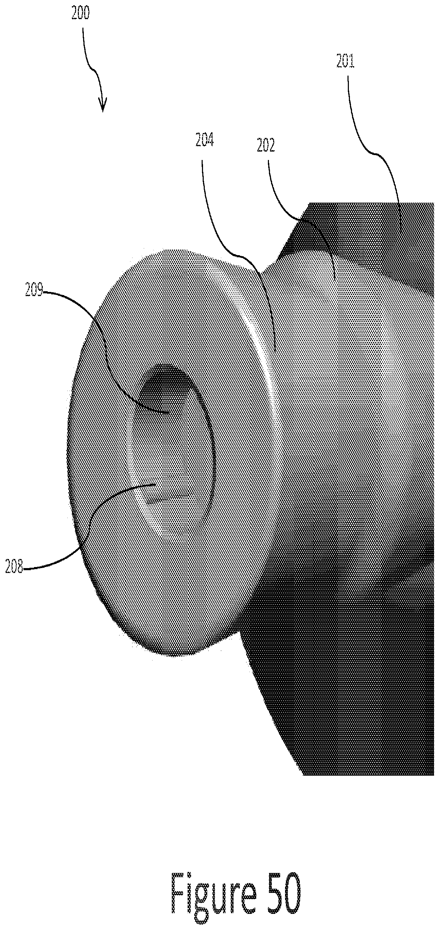

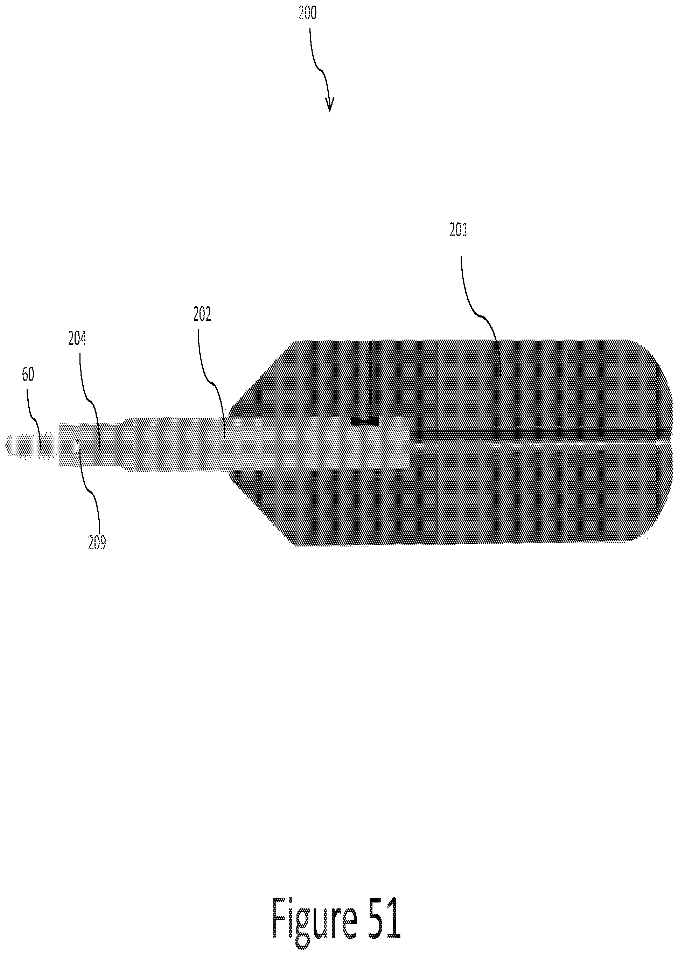

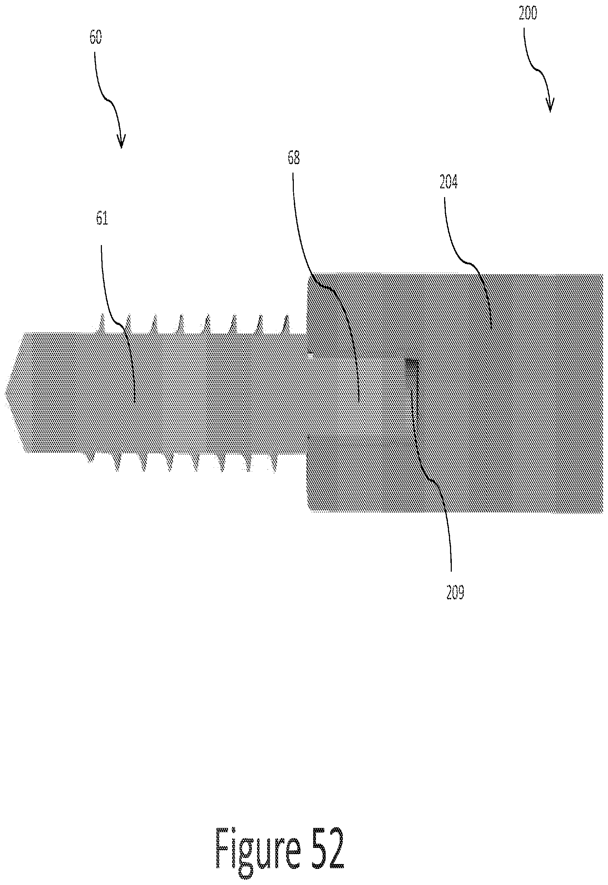

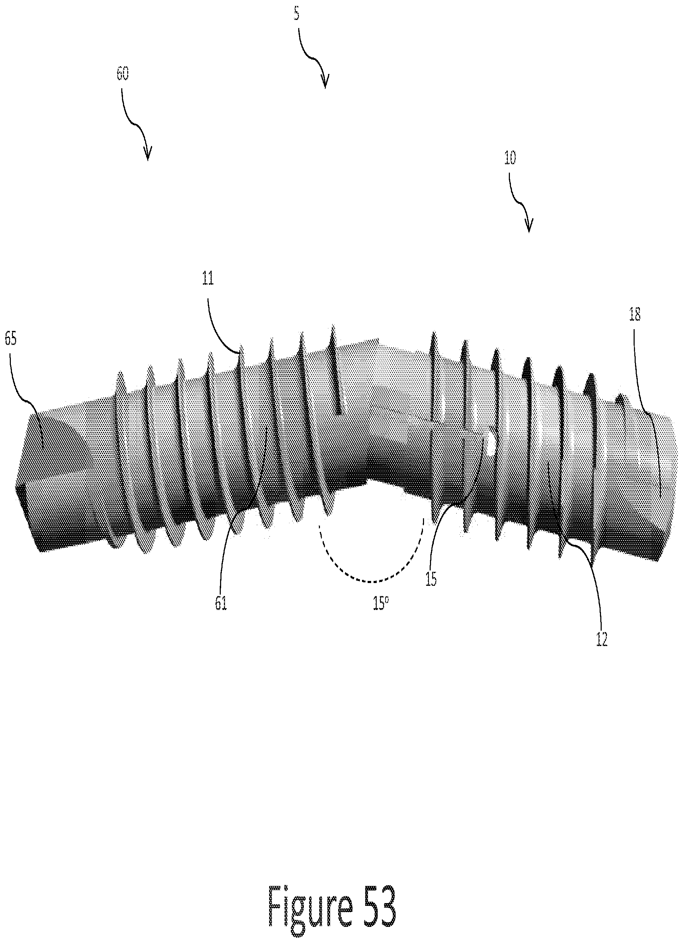

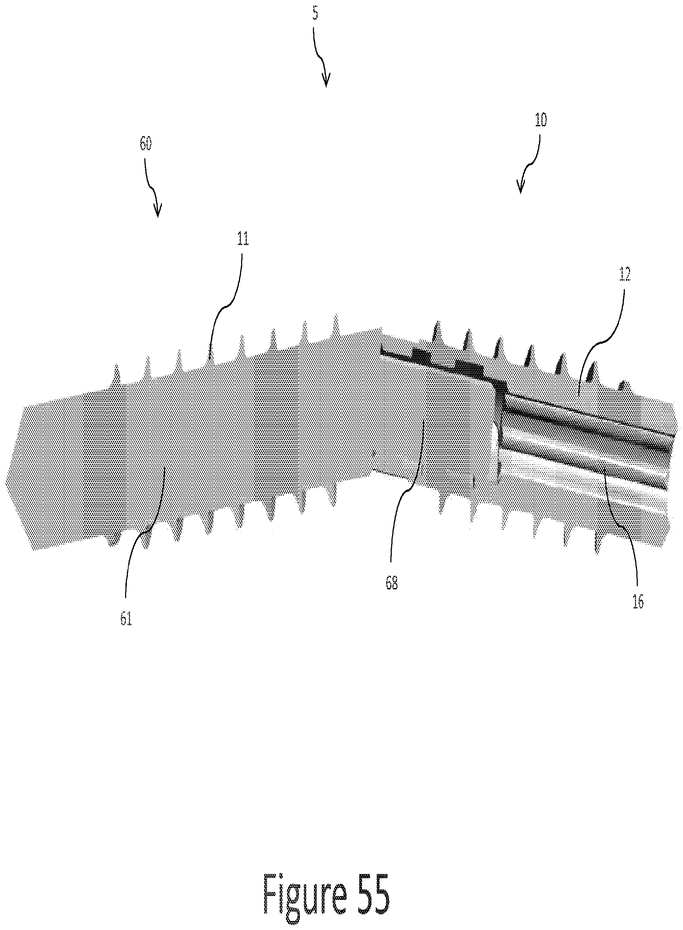

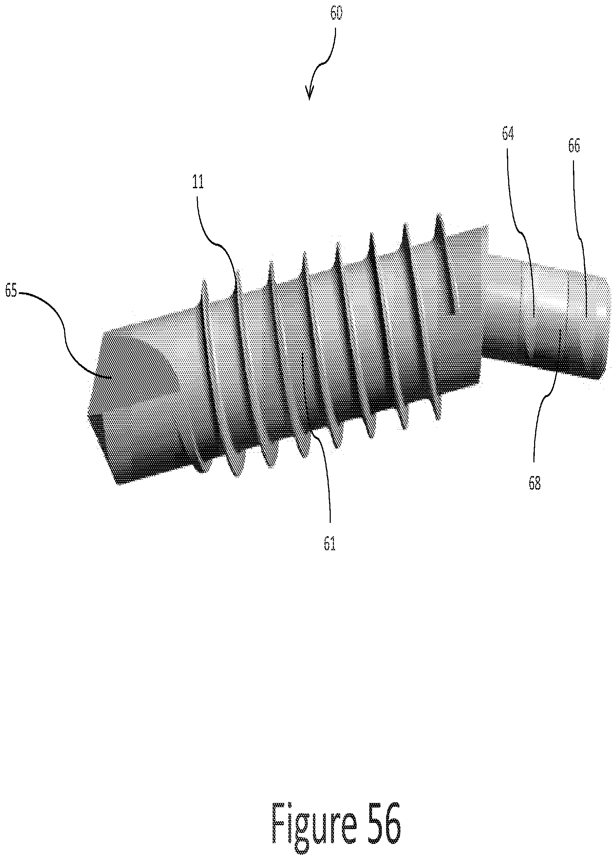

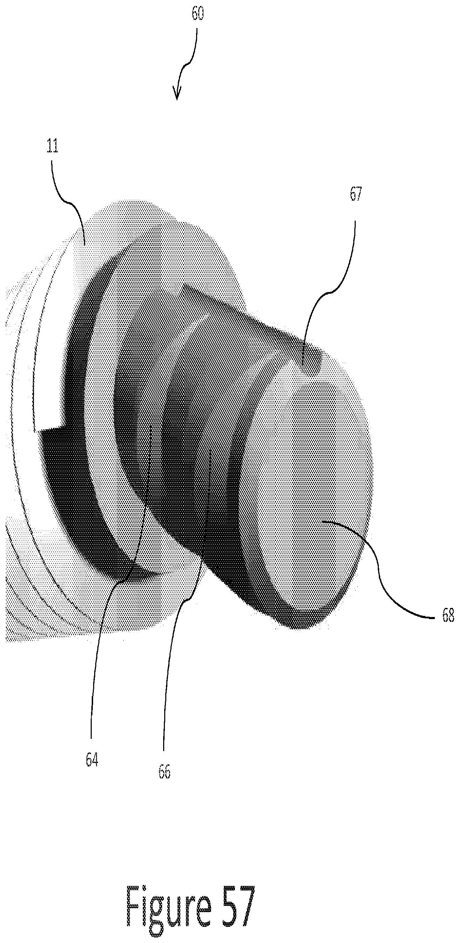

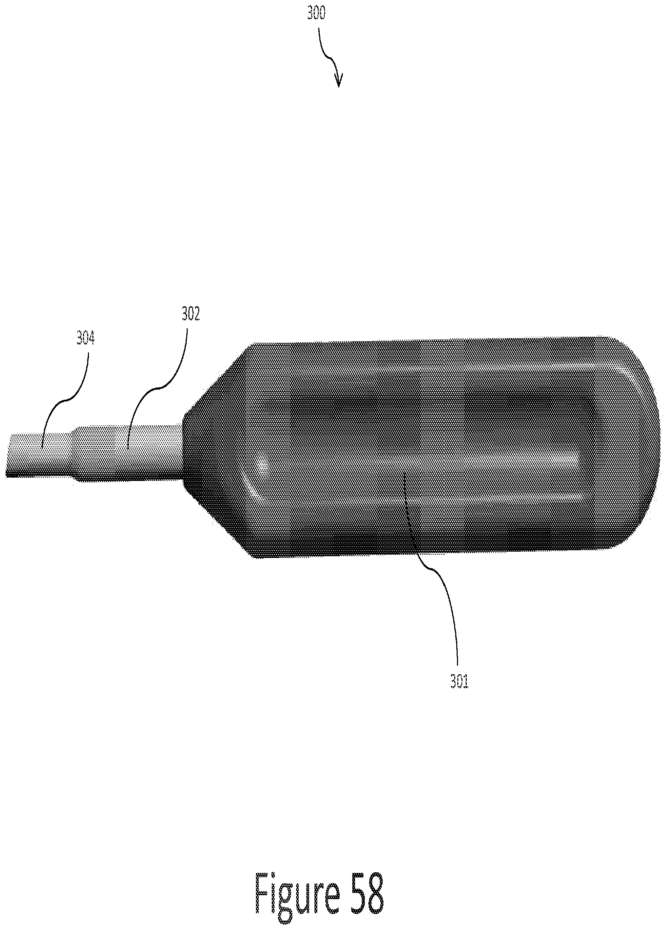

FIGURES 1-21 depict a first embodiment of the PIP fusion apparatus. FIGURES 22-24 depict a cross-section of the attachment assembly; FIGURES 25 -29 depict an embodiment of the delivery tool; FIGURES 30-34 depict an embodiment of the compressor tool; FIGURE 35 depicts a partial section of a human foot with the PIP fusion apparatus installed at the PIP joint between the proximal and medial phalanges of one of the lesser toes. FIGURES 36-40 depict an alternate embodiment of a method for installing the PIP fusion apparatus. FIGURES 41-44 show an alternate compressor tool having a ball-headed Allen Wrench head alone and engaging the compressor lumen. FIGURES 45-48 show a linear 2-piece PIP fusion device. FIGURES 49-52 show the corresponding delivery tool. FIGURES 53-57 show a bent 2-piece PIP fusion device. FIGURES 58-59 show the corresponding delivery tool.
FIGURES 1 and 2 are horizontal perspective computer-designed figures of the assembled PIP fusion device with compressor portion on the left and the anchor portion on the right; FIGURE 2 is the same picture but oriented pointing away from the viewer at approximately a 45 degree angle, thereby showing the interior of a portion of the compressor lumen.
FIGURES 3 and 4 are differing perspectives of exploded horizontal computer-designed figures of the three separate components of the PIP fusion device, e.g., the anchor portion on the left, the coupler immediately below, and the compressor portion immediately behind and to the right. FIGURE 4 is tilted downwards at a 45 degree angle to give a slightly different perspective.
FIGURES 5 and 6 are computer-aided horizontal perspective pictures of the anchor portion.
FIGURE 7 is a slightly off-center top-down view of the anchor portion.
FIGURE 8 is an end-down view of the anchor portion.
FIGURE 9 is two slightly inclined views of the anchor portion from different perspectives so that all of the facets are visible.
FIGURE 10 is an inclined view of a computer-aided picture of the coupler portion of the apparatus.
FIGURE 11 is a more inclined view of a computer-aided picture of the coupler portion of the apparatus.
FIGURE 12 is a view of the coupler along the plane of rotation of the coupler-anchor interface.
FIGURE 13 is a similar view but rotated approximately 45 degrees to reveal additional features of the coupler.
FIGURE 14 is two slightly inclined views of the coupler portion from different perspectives so that all of the facets are visible.
FIGURE 15 is a horizontal view of a computer-aided picture of the compressor portion.
FIGURE 16 is a similar horizontal view but rotated so that a view down the lumen of the compressor is shown.
FIGURES 17 and 18 are compressor first end and compressor second end views, respectively, of the compressor lumen.
FIGURE 19 is two slightly inclined views of the compressor portion from different perspectives so that all of the facets are visible.
FIGURE 20 is a cross-sectional isometric view of an exploded attachment assembly region.
FIGURES 21 and 22 are cross-sectional isometric views of an assembled attachment assembly region, Fig. 21 in unlocked configuration, Fig. 22 in locked configuration.
FIGURES 23 and 24 are horizontal isometric views of a flexed or bent flexible bone fusion apparatus, Fig. 23 in full external view and Fig. 24 a cross-sectional view through the long axis.
FIGURE 25 is a horizontal view of a computer-aided picture of a delivery tool of the invention.
FIGURE 26 is an extreme close-up view of a delivery tool rotated so that the loading shaft lumen is visible.
FIGURE 27 is a horizontal view of a delivery tool with the anchor portion loaded into the loading shaft.
FIGURE 28 is a close-up view of the loading shaft of a delivery tool with the anchor portion loaded into the loading shaft lumen.
FIGURE 29 is a close-up cross-sectional view of the loading shaft of a delivery tool with the anchor portion loaded into the loading shaft lumen.
FIGURE 30 is a horizontal view of a computer-aided picture of a compressor tool of the invention, including both the compressor driver shaft and the compressor handle.
FIGURE 31 is an exploded view of a compressor tool of the invention, including both the compressor driver shaft and the compressor handle.
FIGURE 32 an extreme close-up view of one end of the compressor driver shaft rotated toward the viewer so that the detent and ridges are visible.
FIGURE 33 is top-down view of the compressor handle.
FIGURE 34 is an end-down view of the compressor handle.
FIGURE 35 is partial sectional view of a human foot with an assembled fusion apparatus installed at the PIP joint between the proximal and medial phalanges of one of the lesser toes.
FIGURE 36 is a close-up view of a delivery tool with an assembled anchor and coupler loaded into the loading shaft lumen.
FIGURE 37 is a close-up cross-sectional view of a delivery tool with an assembled anchor and coupler loaded into the loading shaft lumen.
FIGURE 38 is a horizontal view of a computer aided picture of a delivery tool with a compressor and compressor driver shaft assembly loaded into the loading shaft lumen.
FIGURE 39 is a close-up view of a delivery tool with a compressor and compressor driver shaft assembly loaded into the loading shaft lumen.
FIGURE 40 is close-up cross-sectional view of a delivery tool with a compressor and compressor driver shaft assembly loaded into the loading shaft lumen.
FIGURE 41 is a computer-aided picture of an alternate compressor driver.
FIGURE 42 is a close-up of the head of the alternate compressor driver.
FIGURE 43 is a close-up computer-aided picture of the alternate compressor driver tool engaging the distal lumen of the compressor.
FIGURE 44 is a cross-section of a figure similar to Fig. 43 except that the entire fusion device is shown engaged with the alternate compressor driver tool.
FIGURE 45 is a computer-aided picture of a linear two-part PIP fusion apparatus.
FIGURE 46 is an exploded view of a linear two-part PIP fusion apparatus.
FIGURE 47 is a cross-sectional view of a linear two-part PIP fusion apparatus.
FIGURE 48 is a close-up angled view of the coupling end of the anchor portion of a linear two-part PIP fusion apparatus.
FIGURE 49 is a computer-aided picture of a delivery tool for a linear two-part PIP fusion apparatus.
FIGURE 50 is a close-up angled view of the loading shaft and lumen of a delivery tool for a linear two-part PIP fusion apparatus.
FIGURE 51 is a cross-sectional view of a delivery tool for a linear two-part PIP fusion apparatus.
FIGURE 52 is a close-up cross-sectional view of the loading shaft and lumen of a delivery tool for a linear two-part PIP fusion apparatus.
FIGURE 53 is a computer-aided picture of a fixed-angle two-part PIP fusion apparatus.
FIGURE 54 is an exploded view of a fixed-angle two-part PIP fusion apparatus.
FIGURE 55 is a cross-sectional view of a fixed-angle two-part PIP fusion apparatus.
FIGURE 56 is a computer-aided picture of the anchor portion of a fixed-angle two-part PIP fusion apparatus.
FIGURE 57 is a close-up angled view of the anchor portion of a fixed-angle two-part PIP fusion apparatus.
FIGURE 58 is a computer-aided picture of a delivery tool for the anchor portion of a fixed-angle two-part PIP fusion apparatus.
FIGURE 59 is a cross-sectional view of a delivery tool for the anchor portion of a fixed-angle two-part PIP fusion apparatus.

### 1. Introduction

The various embodiments of the present invention provide a system, including apparatus and kits for connecting bones and/or bone portions using a multi-part bone connector with one or more rotating joints. One problem in the art being addressed by the embodiments of the present invention is that of fusing two bones or fragments of a single bone that need to be fused in a non-linear manner, e.g., fusion of the proximal and medial phalanges, or of the proximal and medial phalanges in the foot in an aligned but slightly fixed angle. Fusion of the proximal interphalangeal ("PIP") joint in the hand to alleviate pain associated with Rheumatoid arthritis is a typical application, or to alleviate the condition known as "Hammertoe" in the foot. A first embodiment of a flexible bone fusion apparatus comprises an anchor portion having an elongated body with retaining elements, typically screw threads, on at least a portion of its exterior, the anchor having a leading tip and an end. The anchor is the leading part of the multi-part bone fusion apparatus and in a PIP application the anchor portion of the apparatus is driven into and resides permanently in the medullary cavity or canal of the proximal phalange. The anchor works in combination with a second component called the compressor portion, which similarly has an elongated body with retaining elements that are typically screw threads that may be but are not necessarily of the same pitch and diameter as those of the anchor. In a PIP application, the compressor portion is separately installed into the medial phalange. There are two separate methods of installation. In a first embodiment the anchor-only is installed into the proximal bone, followed by a compressor-coupler combination in the medial bone. In this embodiment of the bone fusion apparatus, the flexible coupler has an interface at each end for interfacing with the anchor and compressor, respectively. In one embodiment the coupler has a first end adapted to rotatably engage the anchor in a first plane with one degree of freedom. The first plane is defined by the two-component rotating attachment assembly shared between the coupler and the anchor. The coupler at its other end ("second end") engages the compressor which is adapted to rotatably engage the compressor through axial rotation. In one embodiment this is accomplished through a ridge-and-annular groove arrangement wherein the coupler shaft snaps into and is longitudinally retained by the compressor, yet they are free to rotate 360 degrees relative to each other around their mutual axis. This allows axial rotational freedom between the coupler and the compressor body which enables compression of the proximal and medial phalanges when the compressor is rotated in reverse. "Axial rotation" is rotation about a transverse axis of the anchor and/or compressor portions as depicted by the rotating arrow in Fig. 1, which shows clockwise axial rotation about the axis of the assembled flexible bone fusion apparatus. In a second installation step, the compressor-coupler combination is advanced compressor first into the end of the medial bone, and after it has reached its final position, the coupler end (which remains outside the bone in the space formerly occupied by the joint) is connected to its mating attachment assembly surface on the anchor, and the two facing bones are now united through the bone fusion apparatus. In a second installation method, the coupler is attached to the anchor on installation, the compressor-coupler unit is then installed, and the compressor and coupler are then connected by inserting the snap-fit interfaces together. The sole difference between the two embodiments is the order of installation of the coupler.

An advantage of this embodiment is that the anchor-coupler attachment region allows rotation in a single plane only, and then is locked in flexed position by counterclockwise rotation of the compressor which pulls the anchor and compressor away from each other thereby locking the flexed orientation. This single-plane limitation is an advantage over prior art apparatus that allow for multiple degrees of freedom, thus allowing unwanted flex off the desired axis during the early healing phase.

### 2. Defined Terms

The terms "anchor," "anchor portion," "anchor element" or "anchor component" are used interchangeably to mean the leading component of a two- or three-component bone fusion apparatus described and taught herein. The second or trailing anchoring element is called by the interchangeable terms "compressor, "compressor portion," "compressor element" or "compressor component." The anchor portion and compressor portion both function as bone anchoring devices. Bone anchoring devices are well-known in general and can take many forms. The anchor components may include two or more discrete anchor elements that can engage bone to resist removal of each anchor element. Each anchor element may be unitary (one piece) or may be formed of two or more pieces, such as two or more pieces that are affixed to one another. The flexible bone fusion apparatus also may include one or more other discrete components, such as one or more discrete spacer components disposed between the anchor elements and/or one or more end components flanking the anchor elements adjacent one or both opposing ends of the connector.

The anchor elements may have any suitable size and shape. The anchor elements may be about the same length (the characteristic dimension measured parallel to the central axis) or different lengths. For example, the compressor element may be shorter or longer than the anchor element. The anchor elements may have about the same diameter or different diameters. The diameter of each anchor element may be generally constant or may vary along the length of the anchor element. For example, the anchor element may taper distally, proximally, or both. Furthermore, the anchor element may have a distal tapered nose (threaded or nonthreaded) that enters bone first.

The flexible bone fusion apparatus may include a spacer region. The spacer region may have any suitable position(s) in the bone fusion apparatus and/or within an anchor element relative to a retention mechanism of the anchor element. For example, the spacer region may be disposed between a threaded region and a flexible joint of an anchor element. The spacer region may be unitary with an associated threaded region (or other retention structure) of an anchor element or may be formed by a distinct component joined fixedly (e.g., welded, bonded, or threadably coupled) or connected movably (e.g., coupled by a movable joint) to the threaded region (or other retention structure). The spacer region(s) may have any suitable length relative to the threaded region (or other retention structure) of an anchor element, including shorter, longer, or about the same length as the threaded region (or retention structure). Furthermore, the spacer region may have any suitable diameter or width relative to the threaded region (or retention structure) and/or protuberance, including a lesser (or greater) diameter or about the same diameter as that of the diameter of the threaded region. The spacer region may, for example, provide a nonthreaded region (and/or a non-anchoring portion of an anchor element(s)) to be disposed at the interface between bone members in which the flexible bone fusion apparatus is installed and/or may help define a range of bending motion of the flexible joint.

The bone fusion apparatus may define any suitable size and shape of lumen or cavity for any suitable purpose. The lumen may extend the entire length of the flexible bone fusion apparatus, such that the apparatus is cannulated, or may, for example, terminate before or after the lumen reaches the leading anchor element and before it reaches the leading end of the flexible bone fusion apparatus. The lumen may have a constant or varying cross-sectional geometry, which may be constant or vary within or compared between anchor elements. In some examples, the lumen may define a driver or delivery tool pattern in both anchor elements, so that a driver may extend through the trailing compressor element and into the leading anchor element, for concurrent engagement and rotation of both anchor elements. In some examples, the lumen may define a driver or delivery tool pattern in both the compressor element and an intervening flexing joint so that a driver may extend through the trailing compressor element and into the intermediate flexing joint, for concurrent engagement and rotation of both compressor and joint elements. In some examples, the lumen may narrow (or end) as it extends distally into the leading anchor element, to provide a shoulder for a tip of the driver to bear against, to facilitate driving the anchor into bone.

The terms "anchor-coupler interface" and "conical two-part single-plane interface" "attachment assembly 20"are used interchangeably to refer to the flexible joint assembly defined by the coupler-anchor interface of one of the present embodiments. The attachment assembly 20 is defined by the two attachment portions 21 and 22 shown in the figures. The word "flex" is used in the same manner as the term "bend" and is intended to convey a non-linear arrangement of the anchor and compressor portions.

Each anchor element may have any suitable retention mechanism. The anchor element may include a retention mechanism that is actuated by placement into bone and/or after placement into bone. The anchor elements of a flexible bone fusion apparatus may have the same type of retention mechanism (e.g., each having an external thread) or may have different types of retention mechanisms (e.g., one having an external thread and another having a nonthreaded engagement with bone).

A retention mechanism that is actuated by placement into bone may be defined by an anchor element that has a cross-sectional dimension (such as diameter) that is larger than the diameter of a hole into which the anchor element is placed. The anchor element thus may be disposed in a friction fit with bone (and/or may cut into bone) as it is placed into the bone. In some examples, the cross-sectional dimension may be defined in part by one or more projections that extend laterally from the body of the anchor element. Exemplary projections may include an external thread, one or more barbs, one or more circumferential ridges, one or more hooks, and/or the like. The projections may be biased (e.g., angled toward the trailing end of the anchor element), to facilitate insertion and to restrict removal. Alternatively, or in addition, the anchor element may have a cross-sectional dimension that increases toward an end (such as a trailing end) of the anchor element (e.g., a flared (e.g., frustoconical) anchor element). Anchor elements that engage bone and resist removal as they are placed into bone may be driven into bone rotationally (e.g., threaded into bone) and/or translationally (e.g., hammered into bone).

A retention mechanism that can be actuated in situ after placement of an anchor element into bone may be provided by expansion/deformation of the anchor element at a selected time after placement. The expansion/deformation may be any change in the structure of the anchor element that increases a cross-sectional dimension of the anchor element at one or more (or all) positions along the placement axis (e.g., the long axis) of the anchor element.

The flexible bone fusion apparatus may be configured as a bone screw having one or more anchor elements ("screw elements") with an external thread. The external thread may have any suitable thread structure. Each screw element may include a single thread (e.g., a continuous rib and/or furrow) or a plurality of threads. The plurality of threads may be disposed in discrete axial regions of the screw element (e.g., spaced proximal and distal threaded regions on the screw element) and/or may share the same axial region (e.g., to produce a multi-threaded configuration). The thread (or threaded region) of each screw element may extend over any suitable portion of the screw element's length, including at least substantially the entire length or less than about half the length, among others. The screw elements preferably have a thread of the same pitch to avoid creating more than one thread channel in the bone, thereby potentially weakening the bone or lessening the ability of the threads to be retained by the bone. The thread may have any other suitable features. For example, the thread (and thus the corresponding screw element) may have a constant or varying major and/or minor diameter within a screw element and/or between the screw elements.

In some embodiments of the flexible bone fusion apparatus, the screw elements, and particularly the leading screw element, may be configured to be self-drilling and/or self-tapping as the bone screw is advanced into bone. For example, a leading tip region of the leading anchor element may include a cutting structure(s) to drill bone, and/or a threaded region of either or both screw elements may include a tap region (such as one or more axial flutes, thread notches or tap faces among others) with a cutting edge(s) to tap bone.

The flexible coupler portion has two ends, one of which interfaces with the anchor and one of which interfaces with the compressor. There are two separate and independent interface designs disclosed herein: one is a snap-fit type interface which allows 360 degree axial rotation of one component relative to the other. The other interface is a two-part conical design which only allows rotation through a single plane from approximately 0 to 30 degrees in either direction. In the embodiments disclosed herein, the design choice made was to locate the snap-fit interface at the compressor-coupler interface. Thus by default the two-part conical interface is located at the anchor-coupler interface.

The anchor elements of the flexible bone fusion apparatus may be connected by interfaces or joints suitable to provide the necessary motion. The joints may be movable between the anchor elements, operating by relative sliding motion (pivotal and/or translational) of apposed joint constituents. The joints may also rotate relative to each other. The joints may operate to restrict complete separation of the anchor elements in the absence of bone, while permitting relative rotational and/or translational motion of the anchor elements. The joints may be a composite connection formed collectively by two or more distinct movable joints.

The joints may permit any suitable relative motion. The joints may permit axial translational motion and/or lateral translational motion, or may substantially restrict either or both of these motions. The joints also or alternatively may permit rotational motion about the long axis and/or about one or more transverse axes of the flexible bone fusion apparatus. The rotational motion about the long axis may be unrestricted (allowing full turns) or restricted to less than a full rotation of the anchor elements. The rotational motion about the transverse axes may be determined by the structure of the rotational joint and/or joint constituents, for example, allowing an angular range of motion, about a selected transverse axis, of at least about 0 degrees and/or no more than about 10, 20, 40, or 60 degrees, among others.

The components may be formed of any suitable biocompatible and/or bioresorbable material(s). Exemplary biocompatible materials include (1) metals (for example, titanium or titanium alloys; alloys with cobalt and chromium (cobalt-chrome); stainless steel; etc.); (2) plastics (for example, ultra-high molecular weight polyethylene (UHMWPE), polymethylmethacrylate (PMMA), polytetrafluoroethylene (PTFE), polyetheretherketone (PEEK), and/or PMMA/polyhydroxyethylmethacrylate (PHEMA)); (3) ceramics (for example, alumina, beryllia, calcium phosphate, and/or zirconia, among others); (4) composites; (5) bioresorbable (bioabsorbable) materials or polymers (for example, polymers of alpha-hydroxy carboxylic acids (e.g., polylactic acid (such as PLLA, PDLLA, and/or PDLA), polyglycolic acid, lactide/glycolide copolymers, etc.), polydioxanones, polycaprolactones, polytrimethylene carbonate, polyethylene oxide, poly-beta-hydroxybutyrate, poly-beta-hydroxypropionate, poly-delta-valerolactone, poly(hydroxyalkanoate)s of the PHB-PHV class, other bioresorbable polyesters, and/or natural polymers (such as collagen or other polypeptides, polysaccharides (e.g., starch, cellulose, and/or chitosan), any copolymers thereof, etc.); (6) bone tissue (e.g., bone powder and/or bone fragments); and/or the like. In some examples, these materials may form the body of an anchor element and/or a coating thereon. The anchor component may be formed of the same material(s) or different materials. Exemplary configurations with different materials may include a coupler formed of metal with leading anchor element formed of a titanium alloy and a compressor made of a polymer; a leading anchor element formed of cobalt-chrome, a coupler formed of metal and a trailing compressor element formed of a bioresorbable material, among others. The choice of materials is generally within the skill of a person having ordinary skill in the art of implantable medical devices.

The flexible bone fusion apparatus of the present teachings may be fabricated by any suitable process(es). For example, the anchor and coupler elements of the apparatus may be formed separately and then connected to one another.

Each anchor element may be formed by any suitable process(es). Exemplary processes include EDM, molding, machining, casting, forming, crimping, milling, and/or the like. Threads or other retention structure on the anchor elements may be formed at the same time as and/or after formation of other portions of the anchor elements.

The anchor elements and coupler may be connected by any suitable process that allows for the necessary movement after connection. Exemplary processes include snap-fitting the coupler into the lumen of the compressor element, to form a rotatable joint. The compressor lumen may have a mouth that is larger than the width of the coupler, so that the coupler, once it is forced past the mouth and engages the annular grooves within the coupler, remains trapped in the lumen. Other exemplary processes include disposing a coupler in a lumen having a lip, and then crimping or otherwise deforming the lip so that the coupler is retained in the lumen.

The flexible bone fusion apparatus may be installed by any suitable methods. Exemplary steps that may be performed are listed below. These steps may be performed in any suitable order, in any suitable combination, and any suitable number of times.

At least two bone members may be selected. The bone members may correspond to different bones or distinct fragments of the same bone, among others. The bone members may be adjacent one another naturally or may be moved so that they are adjacent one another. The bone members may have sustained or be associated with any suitable injury. For example, the bone members may result from an injury to bone (such as a fracture and/or an osteotomy, among others) or may be adjacent and/or connected to injured soft/connective tissue (e.g., ligament, tendon, and/or muscle, among others). In some examples, the bone members may be bones that articulate with one another through an anatomical joint. Any suitable anatomical joints may be selected, including the scapholunate joint, the acromioclavicular joint, DIP or PIP joints, etc. Any suitable adjacent bones may be selected, including bones of the hand (e.g. phalanges), wrist (e.g., carpal bones), arm, foot, ankle, leg, shoulder, etc.

A bone fusion apparatus may be selected. The bone fusion apparatus may have any combination of the features described elsewhere in the present disclosure including having a flexible joint. Furthermore, the bone fusion apparatus may have a size (e.g., length and width) selected according to the size of the bone members into which the bone fusion apparatus is to be placed (e.g., a narrower and/or shorter bone fusion apparatus for smaller bone members and a wider and/or longer bone fusion apparatus for larger bone members).

The flexible bone fusion apparatus may be placed into a pre-formed hole in the bone members. The hole may be formed, for example, by drilling through the proximal bone member and into the distal bone member (or vice versa). In some examples, the hole may be formed by drilling over a wire placed into the bone members, to define a guide path along which a drill and the flexible bone fusion apparatus travel. Accordingly, the drill and/or flexible bone fusion apparatus may be cannulated so that each can slide along the wire. Alternatively, the flexible bone fusion apparatus (and particularly a flexible bone screw) may be self-drilling so that it forms and/or widens its own hole as it advances into bone.

The flexible bone fusion apparatus may be left in place permanently or may be removed at a later time. Removal of the flexible bone fusion apparatus may take place at any suitable time. Exemplary times include at a predefined time or after a predefined amount of healing. In some examples, the flexible bone fusion apparatus (and/or an anchor element thereof) may be bioresorbable, so that the flexible bone fusion apparatus (and/or an anchor element thereof) is broken down by the body over time.

### 3. Examples

The following examples are illustrations of selected embodiments of the inventions discussed herein, and should not be applied so as to limit the appended claims in any manner.

### Example 1 -Flexible Three-part PIP Fusion Apparatus

Figures 1-21 disclose a first embodiment of the invention. The flexible bone fusion apparatus 5 of Figure 1 comprises three main components: a compressor portion 10, coupler portion 30, and anchor portion 60. The coupler portion imparts overall flexibility to the design and allows the bone fusion apparatus to flex in a single plane at a PIP joint to emulate a natural curvature of the joint (e.g., finger or toe) post-fusion. Figure 1 depicts all three components together in their unlocked assembled arrangement. Figures 25-34 show a delivery tool 100 and a compression tool 110 that are both similar to a conventional screwdriver in appearance and function. The delivery tool 100 is used to install the components into bone, and the compression tool 110 is used to compress the bones to be fused after the entire apparatus 5 is installed.

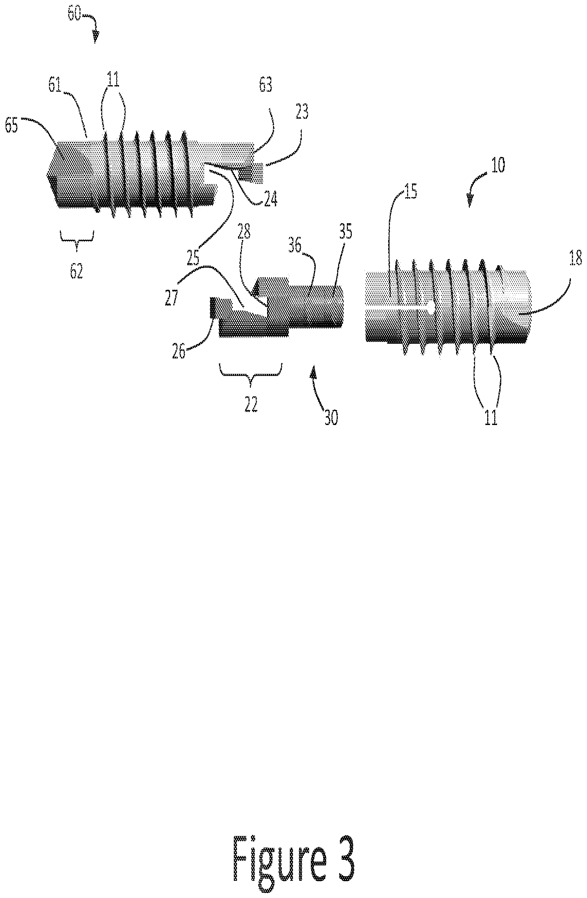

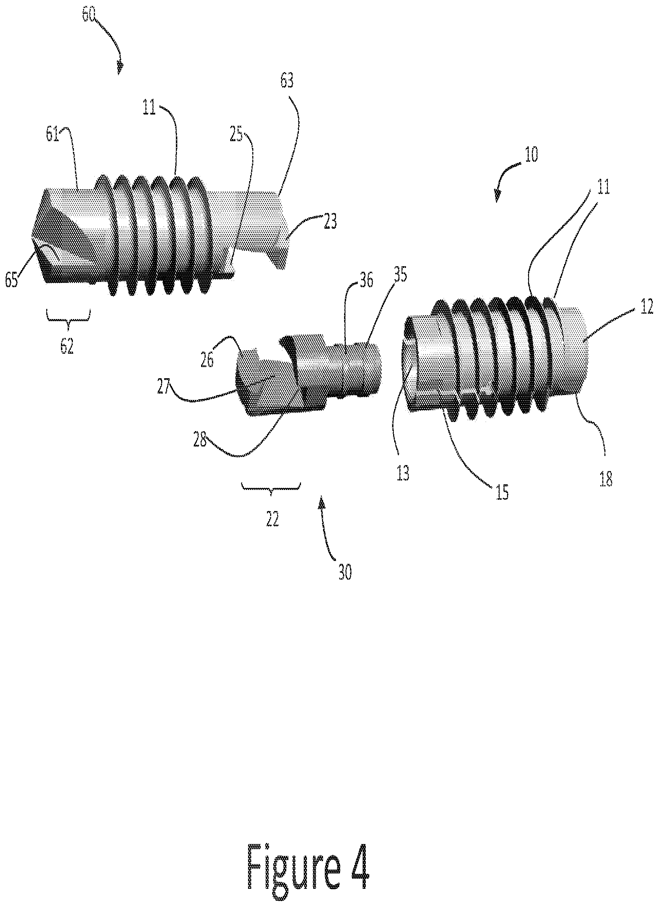

Figures 3 and 4 show an exploded horizontal perspective figure of the bone fusion device. Anchor portion 60 has an anchor body 61 that is made from any material suitable for medical implantation such as stainless steel, titanium, or a biologically compatible polymer. The main requirements are that the material be strong yet light, be sanitizable, and be able to withstand the usual rigors of installation. A preferred embodiment is made from titanium alloy. The embodiments shown throughout this description are made via Electric Discharge Machining, (EDM) a technique well-known in the machining arts, in combination with lathe cutting, grinding, abrasion and polishing. The dimensions of the anchor body are from about 10 mm to about 40 mm in length, and a diameter from about 2 mm to about 5 mm. Disposed upon at least a portion of the anchor body 61 are screw threads 11. In the first embodiment the threads are of a uniform diameter and pitch (distance between adjacent threads), although the leading tip of the anchor may also utilize a screw thread having a smaller leading diameter to initially engage the bone. A typical thread diameter ranges from about 2.5mm to about 6mm. Thread diameters and pitch will of course vary depending upon the desired application.

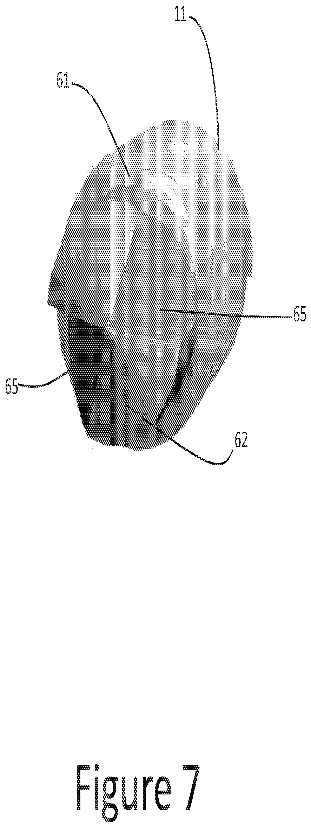

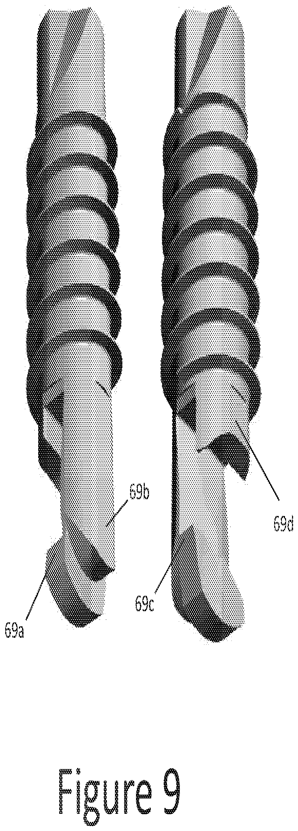

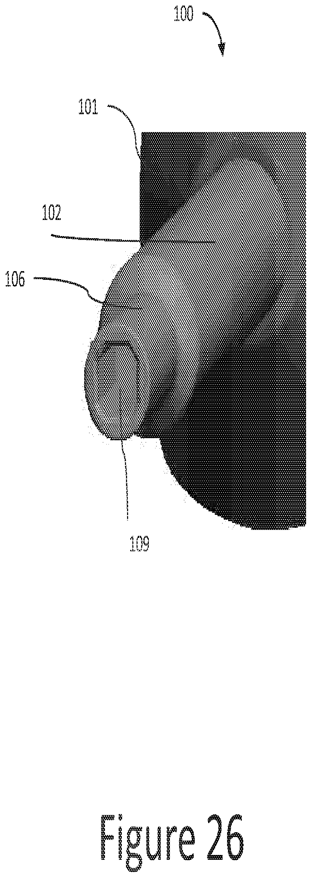

Anchor portion 60 also features a plurality of facets 69a-d around the circumference of its anchor end 63 (Fig. 9). The facets are arranged in a pattern that matches a driving pattern in the delivery tool 100 and allows the anchor portion 60 to fit securely within the loading shaft lumen 109 of driving tool 100 (Fig. 26). The facets will typically each comprise a flat face, though other geometric conformations may also be used. A minimum of three facets is required to achieve a secure fit. A preferred embodiment utilizes four facets spaced evenly around the circumference of the anchor component 60; however, additional facets and alternate spacing arrangements may be used if desired. In a preferred embodiment one of the four facets is different and matches a complementary difference in the driving pattern of the driving tool so that the tool and anchor portion can be "clocked." Clocking the patterns allows the alignment mark on the driving tool to be aligned with the top or dorsal side of the medial phalange thereby alerting the installing surgeon to the correct orientation of the anchor. Anchor portion 60 has a leading tip 62 that has at least one anchor tap edge 65 useful for cutting through bone (Fig. 7). The anchor tap edge 65 is cut from the conical tip and body such that when the tip is rotated in contact with bone the cutting surface will engage the bone and carve the bone away at the leading edge. The conical portions of the leading tip 62 have utility for guiding the apparatus into the medullary canal of the bone, if the application is such that the area of bone being targeted for repair has a canal. In some applications a channel through the bone is drilled to act as a guide for the bone fusion apparatus and so the anchor tap edge 65 may not come into contact with bone during the installation process unless the channel is too narrow or short.

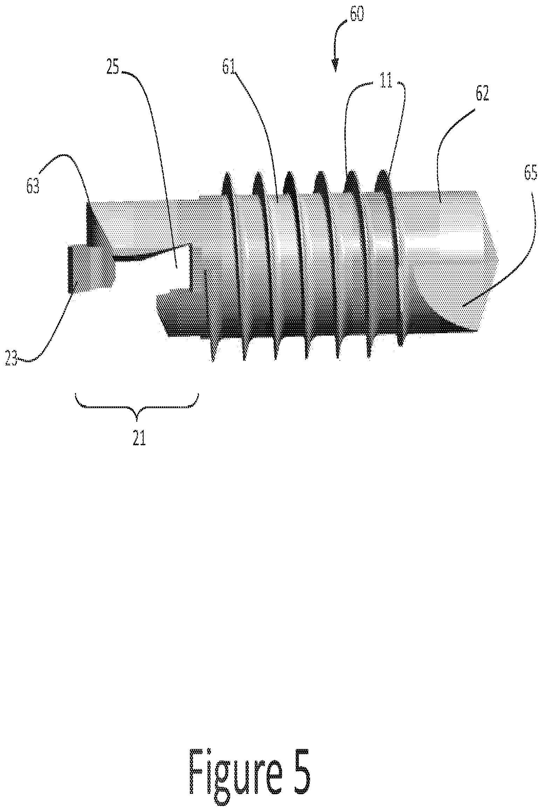

The end of the anchor portion 60 that engages the coupler 30 is the anchor attachment portion 21, seen best in Figs. 5-6 and Fig. 22. Anchor attachment portion 21 has three main features for facilitating rotating attachment to coupler attachment portion 22 of coupler 30. The first feature is the anchor locking wedge 23. Anchor locking wedge 23 is an irregularly-shaped element that fits into annular groove 28 of coupler attachment portion 22. It has three main functions: to hold the top of the anchor attachment portion 21 within the coupler annular groove 28; when unlocked, to facilitate rotation in a single plane; and when installed to lock the angled configuration of the bone fusion apparatus in place. Anchor locking wedge 23 has a greater thickness at the anchor locking wedge edge 73 that engages with the opposing coupler groove bottom 41, as shown in Fig. 22, and so it forms a wedge-shape that is retained in coupler annular groove 28 due to the restrictive dimensions of the coupler groove. (Coupler locking wedge edge 43 is similarly designed to be thicker at its periphery than its internal margin thereby to be retained in complementary anchor annular groove 25.)

The second feature of anchor attachment portion 21 is the anchor annular groove 25, which like the coupler annular groove 28 is designed to accept and retain the locking wedge from the complementary attachment portion. Anchor annular groove 25 is defined by the anchor body 61 on one side, specifically the anchor groove inner wall 70, the anchor groove bottom 71, and the lower portion of the anchor axial face 24 on the side opposing the anchor body 61, the anchor groove semi-circular conical face 72. It will be noted that anchor groove semi-circular conical face 72 is not parallel to its opposing wall, but declines at approximately a 10 degree angle to provide a means for creating a friction face when the coupler and anchor are pulled apart. The 10 degree angled surface defines a partial cone in three dimensions, and the surface of the anchor groove semi-circular conical face 72 is therefore termed "conical," although the apex of the cone, if present, would inhabit the empty space immediately above the axial face 24. It should be noted that other surfaces may also serve the function, including a spherical surface.

The third feature of anchor attachment portion 21 is the anchor axial face 24. The anchor axial face is a flat area that defines the rotational axis about which both attachment portions rotate, the center of the axis denoted by the dashed lines in Figures 22-24. The area is flat so that the inner portions of the locking wedges may slip without constraint towards the center thereby allowing the locking function. The anchor groove semi-circular conical face 72 feature functions to center the opposing faces during rotation.

Complementary coupler attachment portion 22 has the same features with the same overall function and design features as the anchor attachment portion, but the features are reversed to complement or fit within the anchor attachment portion features. In this way they function together as a two-part interface to rotate about the single plane defined by the semi-circular conical faces located at the center of the attachment assembly 20. As previously mentioned, the anchor locking wedge 23 fits within the coupler annular groove 28 because the groove is machined to have a similar shape for receiving and holding the wedge, but in a manner that allows some amount of "slip" along the longitudinal axis of the flexible bone fusion device.

Coupler attachment portion 22 has three main features for facilitating rotating attachment to anchor attachment portion 21 of anchor 60. The first feature is the coupler locking wedge 26. Coupler locking wedge 26 is an irregularly-shaped element that fits into anchor annular groove 25 of anchor attachment portion 21. It has three main functions: to hold the top of the coupler attachment portion 22 within the anchor annular groove 25; when unlocked, to facilitate rotation in a single plane; and when installed to lock the angled configuration of the bone fusion apparatus in place. Coupler locking wedge 26 has a greater thickness at the edge 43 that engages with the opposing anchor groove bottom 71, as shown in Fig. 22, and so it forms a wedge-shape that is retained in anchor annular groove 25 due to the restrictive dimensions of the anchor groove.

The second feature of coupler attachment portion 22 is the coupler annular groove 28, which like the anchor annular groove 25 is designed to accept and retain the locking wedge from the complementary attachment portion. Coupler annular groove 28 is defined by the coupler body 37 on one side, specifically the coupler groove inner wall 40, the coupler groove bottom 41, and the lower portion of the coupler axial face 27 on the side opposing the coupler body 37, the coupler groove semi-circular conical face 42. It will be noted that coupler groove semi-circular conical face 42 is not parallel to its opposing wall, but inclines at approximately a 10 degree angle to provide a means for creating a friction face when the coupler and anchor are pulled apart. The 10 degree angled surface defines a partial cone in three dimensions, and the surface of the coupler groove semi-circular conical face 42 is therefore termed "conical," although the apex of the cone, if present, would inhabit the empty space immediately above the axial face 27. It should be noted that other surfaces may also serve the function, including a spherical surface.

The third feature of coupler attachment portion 22 is the coupler axial face 27. Coupler axial face 27 is a flat area that defines the rotational axis about which both attachment portions rotate, the center of the axis denoted by the dashed lines in Figures 22-24. The area is flat so that the inner portions of the locking wedges may slip without constraint towards the center thereby allowing the locking function. The coupler groove semi-circular conical face 42 feature functions to center the opposing faces during rotation. After manufacture of the compressor portion and coupler portion the two parts may be assembled by hand. This involves aligning the anchor attachment 21 and coupler attachment 22 portions so that they face each other as in Figs. 3-4 and/or 22-24 and rotating them to approximately 69 degrees off-axis. Since this embodiment has been designed to assemble and function at this angle, they will fit into each other's complementary features. Rotation to 0 degrees will assemble them for further post-assembly steps such as cleaning, sterilization and packaging.

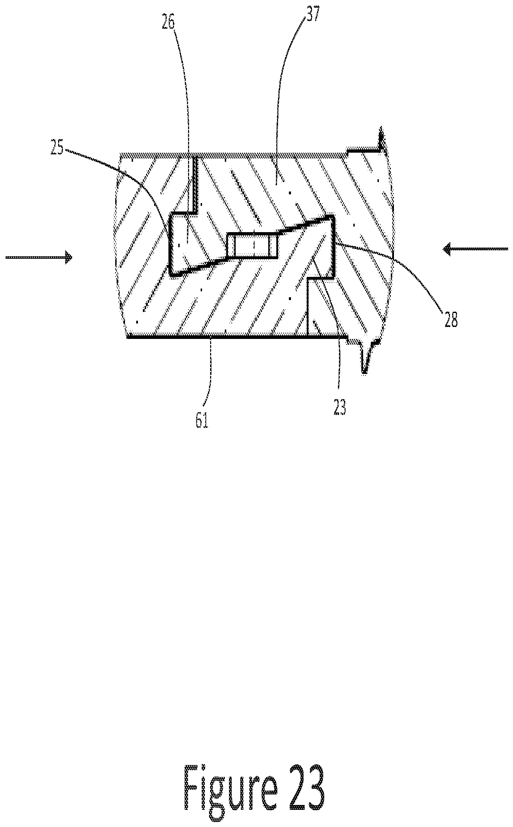

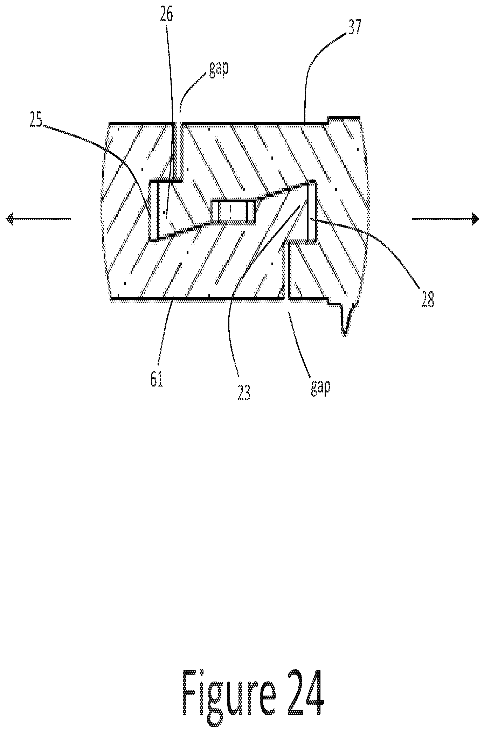

With reference to Figures 23-24, Figure 24 is a cross-section of the assembled PIP fusion device in the locked configuration. There are gaps at the peripheries between the coupler body 37 and the anchor body 61, indicating that the two components have been pulled apart along their longitudinal axis (in the direction of the arrows), thus driving the upper locking wedge portions 23, 26 of both devices into their respective annular grooves 28, 25. In the configuration shown in Fig. 24, friction between the interlocking wedges and mating groove faces constrains further rotational motion after they have been pulled apart in this manner. Figure 23 shows the same components in their unlocked configuration where the components have been pushed together (in the direction of the arrows) thereby freeing the wedges from the grip of the grooves and allowing rotational motion in the plane defined by the axial faces 24 and 27. Thus both the anchor annular groove 25 and the coupler annular groove 28 have groove depths that exceed the length of the corresponding locking wedges 26, 23 enough to facilitate the locking function. In this embodiment, the depths are typically from about 0.75 mm to about 1.5 mm.

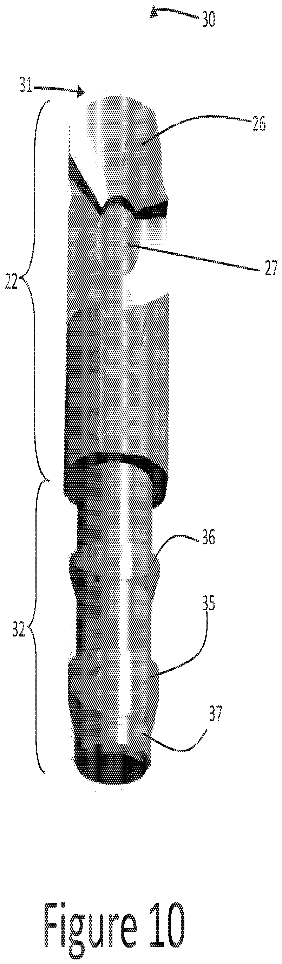

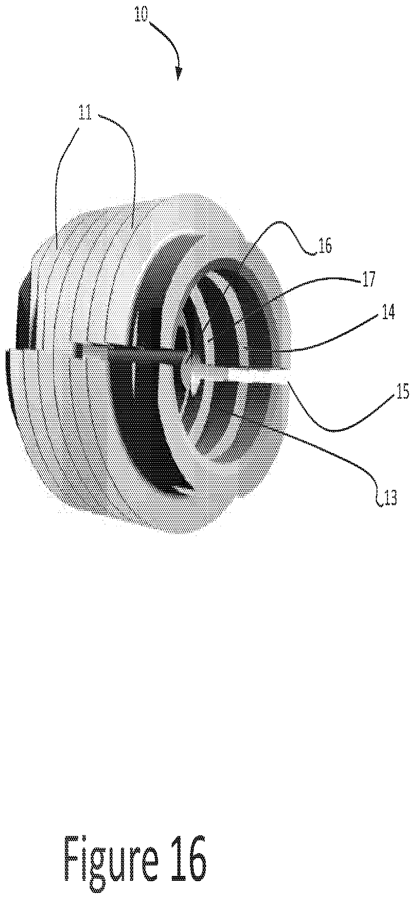

Now with reference to Figs. 10-13, an embodiment of coupler portion 30 includes two main areas of interest, the uppermost coupler attachment portion 22 and the lower half which comprises an axial interface for rotational attachment to the compressor portion 10. As previously described with reference to the anchor attachment portion 21, the three main features for facilitating rotating attachment to anchor attachment portion 21 include a coupler locking wedge 26, a coupler axial face 27 and a coupler annular groove 28. Since the coupler attachment portions have been designed to be complementary to each other, the features have the same general design and function as the features of the anchor attachment portion and are only briefly mentioned. In addition to the three elements of the coupler attachment portion 22, coupler 30 has a coupler first end 31 which is where the coupler locking wedge 26 is located. There is also a coupler second end 32, located at the lower half of the coupler in Figs. 10-13. The coupler second end comprises a coupler body 37 that is roughly cylindrical in shape and has an external diameter which is sized to fit within compressor portion 10. In this embodiment, the external diameter of the coupler is 2.7mm. The coupler body 37 has at least one annular ridge 35 disposed upon its surface for engaging with mating compressor annular groove 14 in compressor 10 (see Fig. 16). In a preferred embodiment coupler body 37 has at least two sets of coupler annular ridges 35 and 36 spaced some distance apart longitudinally for engaging in snap-in fashion with the at least two annular grooves 14 and 17 of compressor lumen 13. Coupler annular ridges 35 and 36 do not have to be continuous, but can be discontinuous shoulder-type ridges as shown in Figs. 10-13.

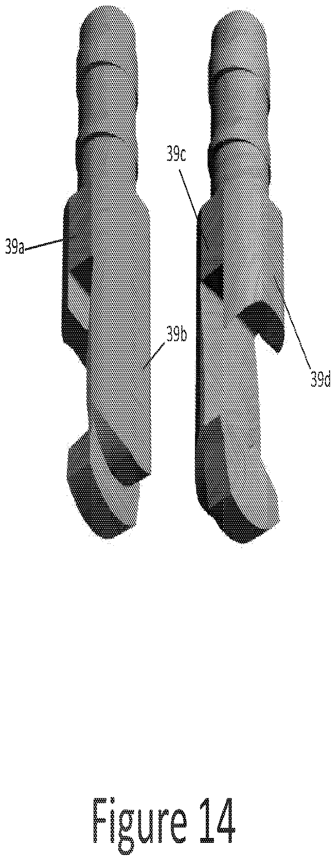

Coupler portion 30 also features a plurality of facets 39a-d around the circumference of its first end 31 (Fig. 14). The facets are arranged in a pattern that matches a driving pattern in the delivery tool 100 and allows the coupler portion 30 to fit securely within the loading shaft lumen 109 of driving tool 100 (Fig. 26). The facets will typically each comprise a flat face, though other geometric conformations may also be used. A minimum of three facets is required to achieve a secure fit. A preferred embodiment utilizes four facets spaced evenly around the circumference of the coupler portion 30; however, additional facets and alternate spacing arrangements may be used if desired.

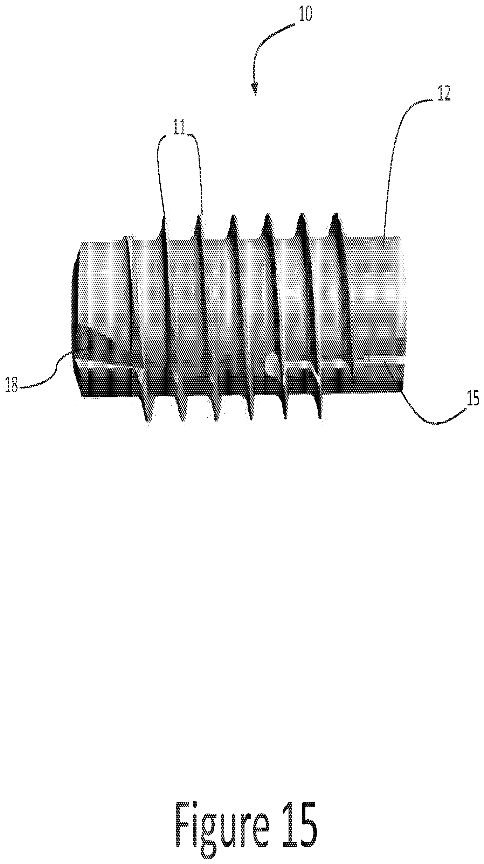

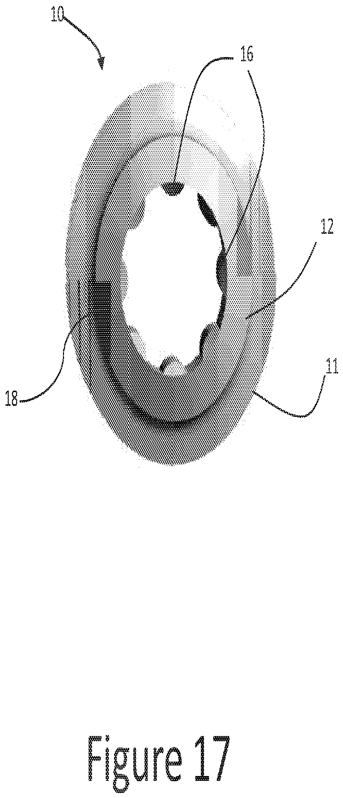

With reference to Figures 15-19, an embodiment of compressor portion 10 is shown. Compressor portion 10 is adapted to engage with coupler 30 at one end, and also to engage with delivery and compressor tools through the compressor lumen 13. In this embodiment, compressor portion 10 is a single piece having a body 12 with retaining means such as threads 11 disposed on its external surface. The threads may have the same pitch and diameter as the threads of the anchor portion, or they may differ from the anchor threads. At least one compressor tap edge 18 that is useful for cutting through bone is also included at one end (Figs. 15, 17). The compressor tap edge(s) 18 is configured such that when the compressor portion 10 is rotated with the compressor tap edge(s) 18 in contact with bone, the cutting surface will engage the bone and carve the bone away at the leading edge. In some applications a channel through the bone is drilled to act as a guide for the bone fusion apparatus and so the compressor tap edge(s) 18 may not come into contact with bone during the installation process unless the channel is too narrow or short.

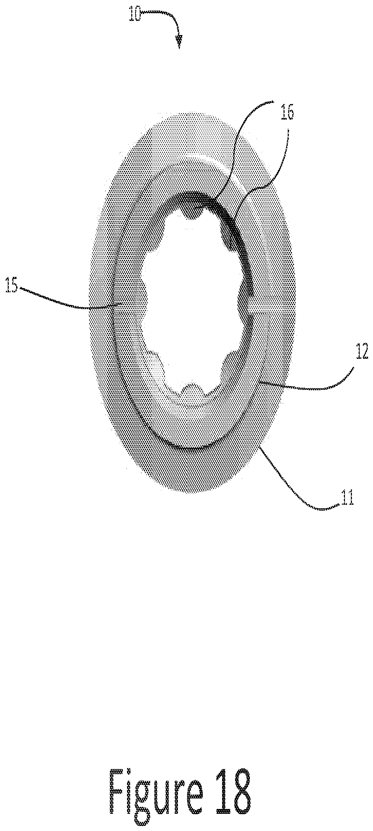

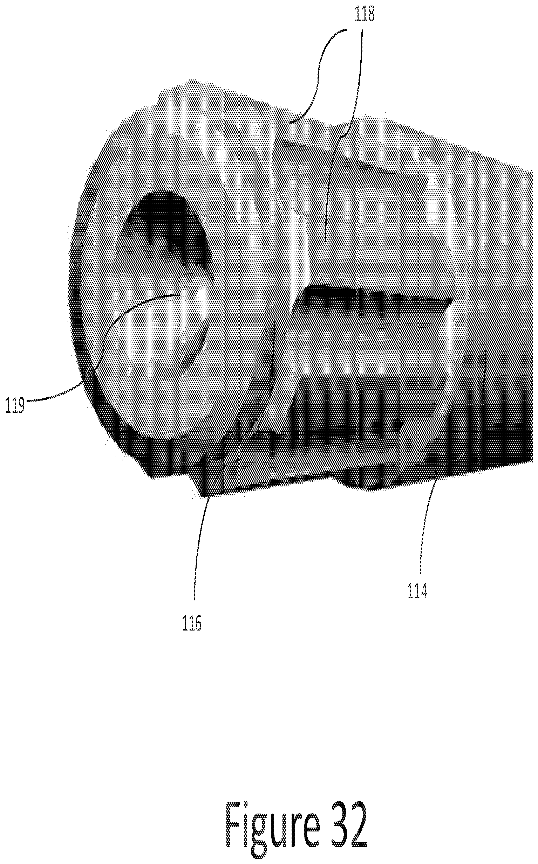

Compressor portion 10 also has a compressor lumen 13 having compressor lumen ridges 16 running lengthwise (or longitudinally) that engage the compressor grooves 118 on Compressor Tool 110. Figures 17 and 18 show eight distinct compressor lumen ridges 16 in cross-sectional perspective. Figure 16 shows that the compressor lumen ridges 16 start at one end of the compressor portion 10 and continue to the region of the compressor annular grooves 14, 17. The length of the ridges should be sufficient to facilitate an adequate grasp of the compressor as the bone fusion assembly is being installed and so their precise length is a design choice. In a preferred embodiment the ridges range from about 5 mm to about 10 mm in length. The eight ridges are spaced octagonally and match the pattern of the eight compressor grooves 118 on the compressor loading shaft 116 of compressor tool 110, as seen best in Fig. 32. This allows a secure fit between the tool and the compressor.

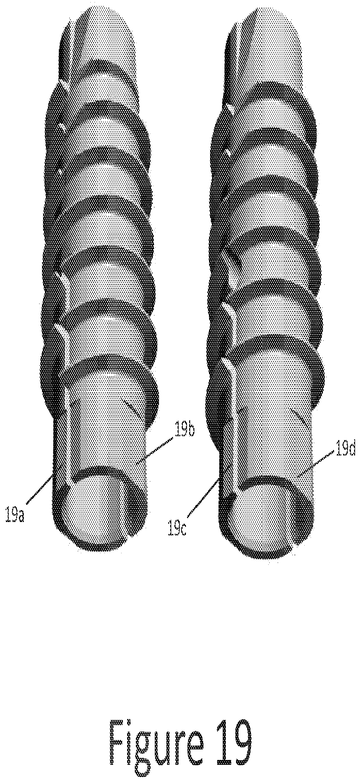

Compressor portion 10 also features a plurality of facets 19a-d around the circumference of the end of compressor portion 10 that attaches to coupler portion 30 (Fig. 19). The facets are arranged in a pattern that matches a driving pattern in the delivery tool 100 and allows the compressor portion 10 to fit securely within the loading shaft lumen 109 of delivery tool 100 (Fig. 26). The facets will typically each comprise a flat face, though other geometric conformations may also be used. A minimum of three facets is required to achieve a secure fit. A preferred embodiment utilizes four facets spaced evenly around the circumference of the compressor portion 10; however, additional facets and alternate spacing arrangements may be used if desired. Alternately, the facets may be located internally, i.e., in the lumen. In a preferred embodiment, the number of facets and spacing arrangement used with compressor portion 10 will be matched to those of anchor portion 60 and coupler portion 30. This allows driving tool 100 to install either an assembly of the anchor portion 60 and the coupler portion 30 or an assembly of the compressor portion 10 and the coupler portion 30 into bone while the two pieces are attached to each other.

For a person having ordinary skill in the art, the detailed implementation of a snap-fit interface is well within his or her skill level. In this embodiment compressor portion 10 has one or more slots 15 cut into and through the body to allow for some radial expansion of the body as the coupler is being press-fit into it. Compressor portion 10 has internal compressor annular grooves 14, 17 cut into the lumen surface to accommodate the coupler annular ridges 35, 36. When the coupler second end (facing the compressor) is pressed into the internal diameter of the compressor, the compressor body will expand slightly and then as the ridges reach the grooves the ridges will snap down into the grooves and stop there. The fit of the grooves and ridges is such that axial rotation is allowed, that is, the diameter at the base of the grooves is close to the external diameter of the ridges. In a preferred embodiment this diameter is about 2.5 mm. In this embodiment, the function of the snap-fit interface in the assembled bone fusion apparatus is to allow free rotation of the compressor portion about the coupler portion. In this embodiment the coupler will be fixed and the compressor will rotate axially to adjust the amount of distance between the two bones or bone fragments. The free rotation at the coupler-compressor interface facilitates compression by insertion of the compressor tool into the compressor lumen, then rotating the compressor portion 10 counterclockwise until the bones contact each other.

Figures 25-29 are computer-generated pictures of a delivery tool 100, which can be used to install anchor portion 60, coupler portion 30, and compressor portion 10 into bone in a manner that is similar to that of a conventional screwdriver. This embodiment comprises a conventional handle 101 adapted to fit the hand of an adult. The handle is attached to a shaft 102 and a loading shaft 106. Shaft 102 is of conventional design and is similar to the shaft of a screwdriver. Loading shaft 106 includes a loading shaft lumen 109 that is designed to fit around the outer circumferences of anchor portion 60, coupler portion 30, and compressor portion 10. The inner circumference of the loading shaft lumen 109 features facets in a pattern that is matched to the facet patterns on anchor portion 60, coupler portion 30, and compressor portion 10. This ensures that each of those portions can fit securely within the loading shaft lumen 109 to facilitate installation of the portions into bone.

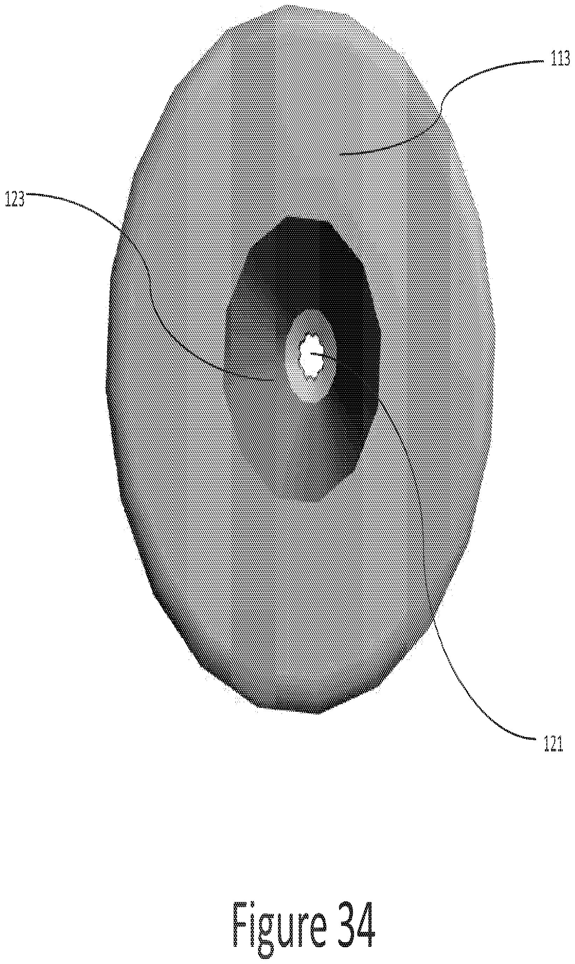

After the anchor and compressor portions have been driven into position in the medial and proximal phalanges, compression of the bones may be desired. To compress the bones a separate tool called a "Compressor Tool" is used. Compressor tool 110 is best seen in Figures 30-34 and comprises a two-part design. Compressor tool 110 includes both a compressor handle portion 111 and a compressor driver portion 112, which may be coupled together or separated from each other. Compressor handle portion 111 is cannulated and includes a handle 113 and a delivery shaft 115. Delivery shaft 115 includes a recessed area 117 with a delivery shaft lumen 121 in its center. The lumen 121 runs the entire length of the delivery shaft lumen and opens into the handle lumen 123 (Fig. 34), thereby creating a cannula that extends through the entire length of handle portion 111. Delivery shaft lumen 121 also includes ridges around its circumference in a pattern that matches that of the lumen ridges 16 in compressor 10. The lumen ridges in both compressor 10 and compressor handle portion 111 create a "driving pattern" that is complementary to a driving pattern on compressor driver shaft 112. This allows the driver shaft to be securely coupled to both the compressor and the handle portion.

Generally, a "driving pattern" is a pattern of grooves, splines, blades or similar machined surfaces that mate with a complementary surface. An example is a TORX® drive, which is a machined shaft having a series of longitudinal grooves that engage a TORX screw or other fitting having a six-sided star-shaped pattern that is designed to accept a TORX driver and nothing else. Other drive patterns include a spline, double hexagonal, hexagonal, tri-wing, triple square, etc. These matching patterns allow compressor driving shaft 112 to fit securely within the lumens of compressor handle portion 111 and compressor portion 10. Compressor tool 110, in its coupled configuration, can then be used to rotate the compressor, thereby facilitating compression of the bones to be fused.

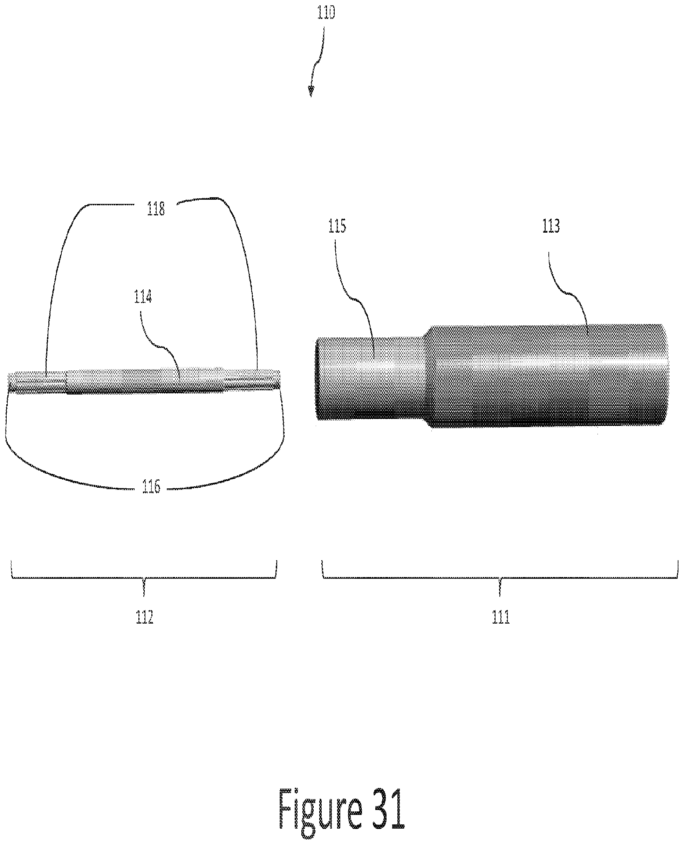

Compressor driver shaft 112 includes a shaft 114 with a loading shaft 116 at each end (Fig. 31). In the illustrated embodiment, the two loading shafts are identical in size and shape. Each loading shaft 116 is designed with an external surface having one or more driving patterns complementary to that of the lumen 13 of compressor 10 and the delivery shaft lumen 121 of compressor handle portion 111. In the embodiment shown in Figure 32, the driving pattern is comprised of eight ridges 118 that are evenly spaced around the external surface of each loading shaft 116. Compressor driver shaft 112 also includes a detent 119 at each end that can accommodate the tip of a segment of K-wire.

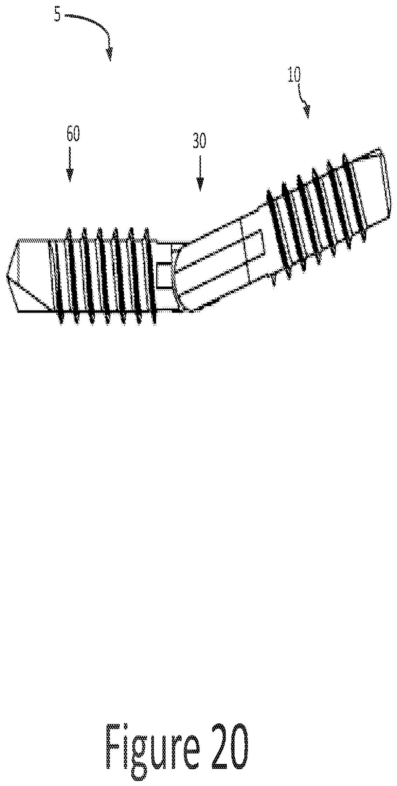

A fully assembled flexed bone fusion apparatus 5 is seen in Figs. 20-21. The range of flex is normally 15 degrees off-axis, however as previously mentioned this particular embodiment is designed to flex as far as 30 degrees and still retain adequate strength at the joint. Both cross-section and external views are shown.

### Example 2 - First Method of Fusing Proximal and Medial Phalanges

Described herein is a method of fusing the proximal and medial phalanges of either a toe or a finger. With reference to the figures, this is accomplished using an embodiment of the inventive apparatus comprising an anchor portion 60, a coupler portion 30, and a compressor portion 10, which together comprise a PIP Fusion Screw 5. First, the correct size Fusion Screw is selected using the x-ray template and pre-op x-rays. Screws sized for either the hand or the foot can be used. A 2 cm dorsal incision over the proximal interphalangeal joint (PIP) joint is then created. The surgeon will then release the collateral ligaments off the head of the proximal phalanx and surgically remove the PIP joint by removing all cartilage from both proximal and medial bones.

The joint can then be trimmed in one of two ways. The first method involves trimming the opposing bone faces flat with both faces having a slight angle relative to the centerline of the bones being connected (0° to 10°) to allow for proper alignment. Alternatively, the surgeon can form ball-and-socket shapes on the opposing faces of the middle and proximal phalanges to allow for alignment adjustments during the surgical procedure. Matched reamer tools can be used to accomplish the resurfacing necessary to accomplish this alternative method.

The surgeon will then center the designated diameter K-wire in the middle of the exposed face of the medial phalanx and drill all the way through the distal interphalangeal joint, continuing through and out of the end of the distal phalanx. Approximately 1-cm of the K-wire should be left exposed from the proximal end of the medial phalanx. The surgeon must then detach the drill and re-attach it at the other end of the K-wire that is protruding out the end of the distal phalanx.

After re-aligning the joint in a straightened orientation, the surgeon will drill the K-wire in the opposite direction so that it inserts approximately 1-cm into the proximal phalanx. The correct positioning of the K-wire can be verified via intraoperative fluoroscopy. The finger or toe should be straight and the K-wire should be centered in both planes. Next, the joint is pulled apart enough to remove the 1-cm portion of the K-wire from the proximal phalanx. The other portions of the K-wire are left in place in the medial and distal phalanges, extending through and out the end of the distal phalanx.

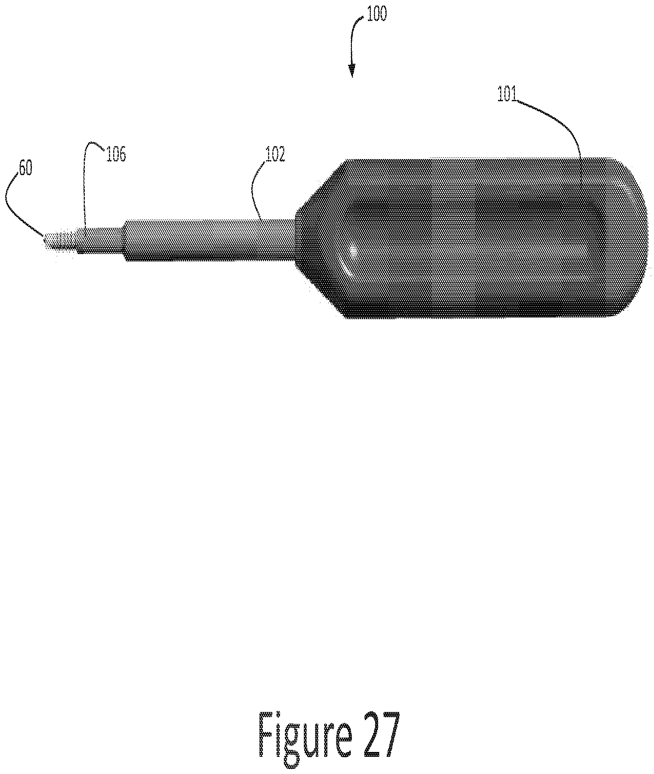

The anchor 60 is then installed into the proximal phalanx. First, it must be loaded into the delivery driver tool 100 so that cutting tip 62 extends away from the tool, as shown in Figs. 27-29. The anchor should then be positioned with its cutting tip at the pilot hole on the face of the exposed proximal phalanx. The delivery driver is then turned clockwise, thereby advancing the anchor into the proximal phalanx, until the anchor attachment portion 21 is located in the correct position relative to the end of the bone. The correct position is apparent when the anchor is as far in as it can go without impinging on the rotation of the joint. To ensure correct rotation of the interlocking joint that will be created by anchor attachment portion 21 and couple attachment portion 22, the surgeon should align the alignment mark on the delivery driver with the dorsal side of the proximal phalanx. The delivery driver is then disengaged and removed from the embedded anchor assembly.

After the anchor is installed, the compressor assembly (compressor 10, coupler 30, and compressor driver shaft 112) is installed into the medial phalanx. First, the assembly must be loaded into the delivery driver 100 with the coupler-compressor loaded first and then the compressor driver shaft. Next, the surgeon aligns the detent 119 at the end of the compressor driver shaft with the tip of the K-wire emerging from the proximal end of the medial phalanx. The surgeon must then press the K-wire towards the end of the distal phalanx with the compressor driver shaft. When the compressor tip engages the medial phalanx, the delivery driver is rotated clockwise, thereby advancing the compressor into the bone until the coupler is located in the correct position relative to the end of the bone. The compressor driver shaft should be left in position, extending through the distal and medial phalanges.

Next, the anchor attachment portion 21 on anchor 60 and the coupler attachment portion 22 on coupler 30 can be hooked together, thereby linking the proximal and medial phalanges. To lock the joint in the desired angular orientation, the surgeon must pull the proximal and medial phalanges apart slightly. If it is necessary to reset the joint angle, the medial and proximal phalanges can be pushed together, thereby unlocking the joint. The phalanges can then be re-aligned for the correct degree of flex. The joint is then re-locked in the desired position by again pulling the proximal and medial phalanges apart. These steps may be repeated until the desired angle is achieved.

Once the device is installed at the correct angle, the proximal and medial phalanges can be compressed to facilitate bone fusion. The surgeon should install the compressor driver handle 111 onto the compressor driver shaft 112 emerging from the distal phalanx, thereby assembling the coupled configuration of compressor driver 110. Then, while holding the distal and medial phalanges, the compressor driver is turned counter-clockwise to draw the proximal and medial phalanges together. The turning of the compressor driver should stop when the resistance increases as the proximal and medial phalanges are compressed together. Intraoperative fluoroscopy can again be used to verify correct positioning of the PIP Fusion Screw 5 and that the bones are in the desired state of compression. Finally, the surgeon should remove the compressor driver shaft and handle, suture the ligament, and close the incisions. An illustration of a Fusion Screw installed in a PIP joint according to this embodiment is shown in Figure 35.

### Example 3 - Second Method of Fusing Proximal and Medial Phalanges

Described herein is an alternate method of fusing the proximal and medial phalanges of either a toe or a finger in which the coupler portion 30 is installed attached first to the anchor portion, rather than to the compressor portion 10 as in Example 2. With reference to the figures, this method also is accomplished using an embodiment of the inventive apparatus comprising an anchor portion 60, a coupler portion 30, and a compressor portion 10, which together comprise a PIP fusion screw or device 5. First, the correct size fusion screw is selected using the x-ray template and pre-op x-rays. Screws sized for either the hand or the foot can be used. A 2 cm dorsal incision over the proximal interphalangeal joint (PIP) joint is then created. The surgeon will then release the collateral ligaments off the head of the proximal phalanx and surgically remove the PIP joint by removing all cartilage from both proximal and medial bones.

The joint can then be trimmed in one of two ways. The first method involves trimming the opposing bone faces flat with both faces having a slight angle relative to the centerline of the bones being connected (0° to 10°) to allow for proper alignment. Alternatively, the surgeon can form ball-and-socket shapes on the opposing faces of the middle and proximal phalanges to allow for alignment adjustments during the surgical procedure. Matched reamer tools can be used to accomplish the resurfacing necessary to accomplish this alternative method.

The surgeon will then center the designated diameter K-wire in the middle of the exposed face of the medial phalanx and drill all the way through the distal interphalangeal joint, continuing through and out of the end of the distal phalanx. Approximately 1 cm of the K-wire should be left exposed from the proximal end of the medial phalanx. The surgeon must then detach the drill and re-attach it at the other end of the K-wire that is protruding out the end of the distal phalanx.

After re-aligning the joint in a straightened orientation, the surgeon will drill the K-wire in the opposite direction so that it inserts approximately 1cm into the proximal phalanx. The correct positioning of the K-wire can be verified via intraoperative fluoroscopy. The finger or toe should be straight and the K-wire should be centered in both planes. Next, the joint is pulled apart enough to remove the 1 cm portion of the K-wire from the proximal phalanx. The other portions of the K-wire are left in place in the medial and distal phalanges, extending through and out the end of the distal phalanx.

Next, the anchor assembly (anchor 60 and coupler 30) is installed into the proximal phalanx. First, the assembly must be loaded into the delivery driver tool 100 with the anchor extending out of the delivery driver tool, as shown in Figs. 36-37. The anchor assembly should then be positioned with its cutting tip 62 at the pilot hole on the face of the exposed proximal phalanx. The delivery driver is then turned clockwise, thereby advancing the anchor into the proximal phalanx, until the interlocking joint between the anchor and the coupler is located in the correct position relative to the end of the bone. The correct position is apparent when the anchor is as far in as it can go without impinging on the rotation of the joint. To ensure correct rotation of the interlocking joint, the surgeon should align the alignment mark on the delivery driver with the dorsal side of the proximal phalanx. The delivery driver is then disengaged and removed from the embedded anchor assembly.

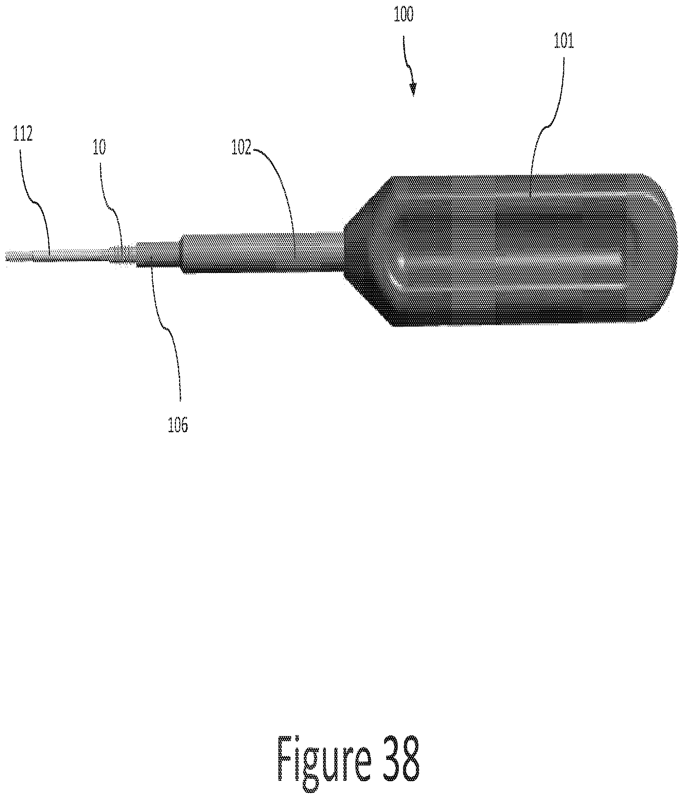

After the anchor assembly is installed, the compressor assembly (compressor 10 and compressor driver shaft 112) is installed into the medial phalanx. First, it must be loaded into the delivery driver 100 with the compressor driver shaft extending away from the delivery driver tool, as is shown in Figs. 38-40. Next the surgeon aligns the detent 119 at the end of the compressor driver shaft with the tip of the K-wire emerging from the proximal end of the medial phalanx. The surgeon must then press the K-wire towards the end of the distal phalanx with the compressor driver shaft. When the compressor tip engages the medial phalanx, the delivery driver is rotated clockwise, thereby advancing the compressor into the bone until the end of the compressor that connects with the coupler 30 is located in the correct position relative to the end of the bone. It should protrude far enough to allow for connection between the compressor and coupler. The compressor driver shaft should be left in position, extending through the distal and medial phalanges.

Next, the coupler 30 can be snapped into the compressor assembly, thereby linking the proximal and medial phalanges. To lock the joint in the desired angular orientation, the surgeon must pull the proximal and medial phalanges apart slightly. If it is necessary to reset the joint angle, the medial and proximal phalanges can be pushed together, thereby unlocking the joint. The phalanges can then be re-aligned for the correct degree of flex. The joint is then re-locked in the desired position by again pulling the proximal and medial phalanges apart. These steps may be repeated until the desired angle is achieved.

Once the device is installed at the correct angle, the proximal and medial phalanges can be compressed to facilitate bone fusion. The surgeon should install the compressor driver handle 111 onto the compressor driver shaft 112 emerging from the distal phalanx, thereby assembling the coupled configuration of compressor driver 110. Then, while holding the distal and medial phalanges, the compressor driver is turned counter-clockwise to draw the proximal and medial phalanges together. The turning of the compressor driver should stop when the resistance increases as the proximal and medial phalanges are compressed together. Intraoperative fluoroscopy can again be used to verify correct positioning of the PIP fusion screw 5 and that the bones are in the desired state of compression. Finally, the surgeon should remove the compressor driver shaft and handle, suture the ligament, and close the incisions.

### Example 4 - Compression via access through Medial Phalange

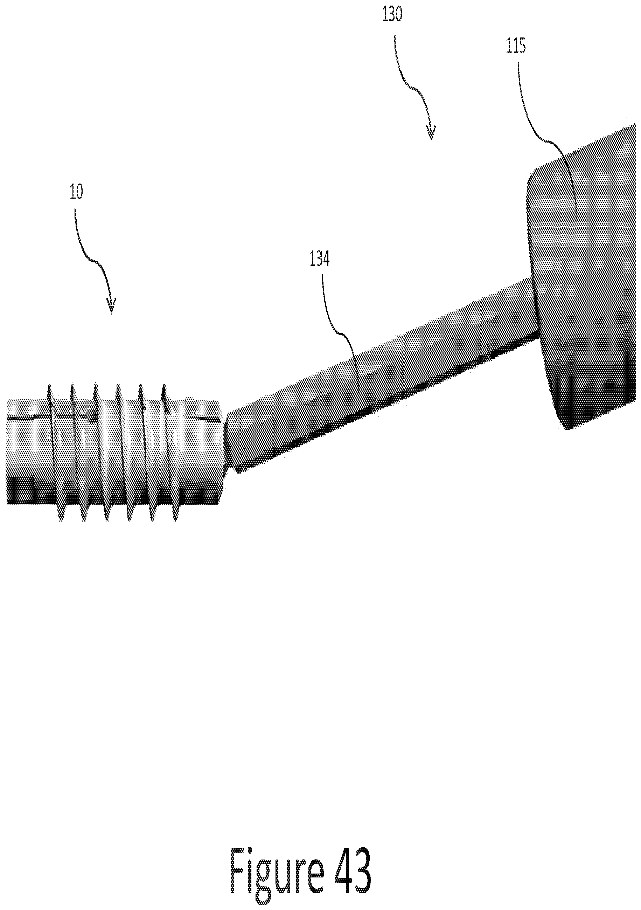

An alternate installation method to those of Examples 2-3 is presented herein. In this embodiment there is no compression driver tool as depicted in Figs. 30-34 and 38-40. Instead of running a K-wire through the distal phalange, no K-wire is used and instead a small access hole or door is cut through the bone on the dorsal side of the medial phalange so that a ball-headed Allen wrench tool may be able to access the open/distal end of the compressor. The distal end of the compressor has an internal hexagonal driving pattern that is engageable by the ball-headed Allen wrench. When the Allen wrench is engaged it may be used to rotate the compressor counter-clockwise thereby moving the bones into compression. Figures 43-44 show a new compressor tool 130 engaged with the end of a compressor.

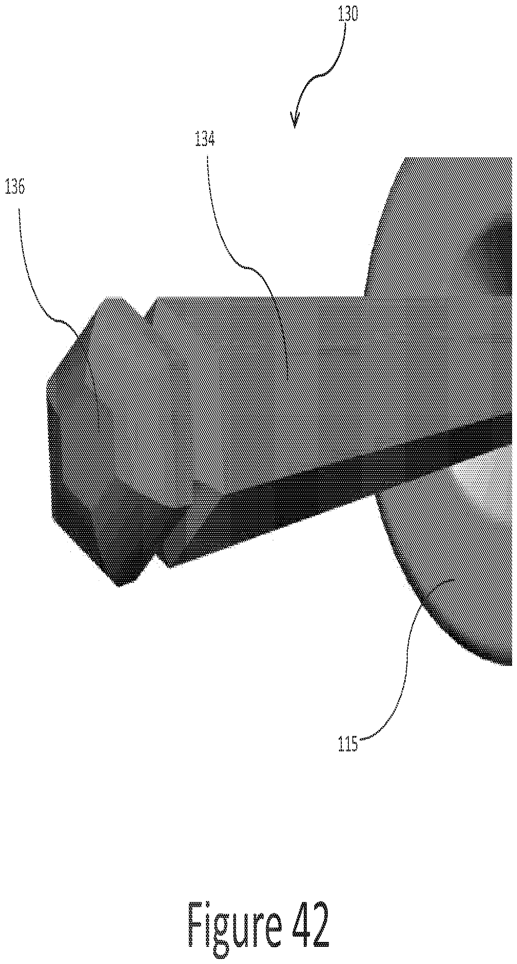

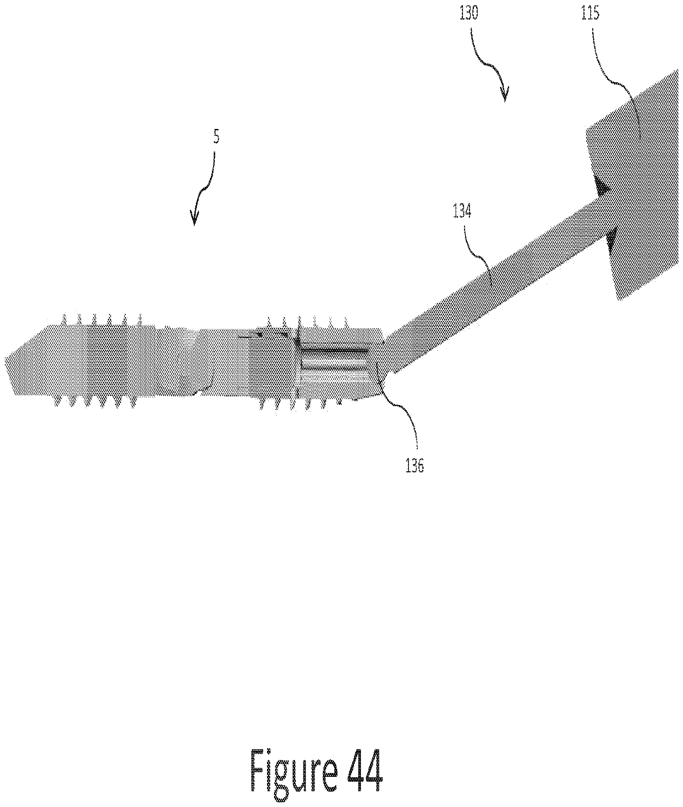

Alternate compressor driver tool 130 is depicted in Figures 41-44. With specific attention to Fig. 41, the tool comprises a handle portion 111 comprising a handle 113 and a delivery shaft 115, and a shaft portion 132 comprising a shaft 134 and a head 136. Shaft 134 may be permanently installed in delivery shaft 115, or it may be a universal delivery shaft into which various shafts may be inserted and removed at will. Fig. 42 is a close-up of the hexagonal ball that will insert into the lumen of the distal end of the compressor, showing the facets of the head 136. The compressor lumen has a driving pattern to complement that of the tool, here a hexagonal pattern. Fig. 43 shows the inserted head 136 in the distal end of the compressor portion 10. Fig. 44 is a sectional view of the device shown in Fig. 43. It is clear that the compressor may be rotated in either direction, clockwise or counterclockwise, merely by turning handle 115 in the desired direction. If counterclockwise, then compression is effected, and vice versa.

### Example 5 - Linear Two-Part PIP Fusion Apparatus

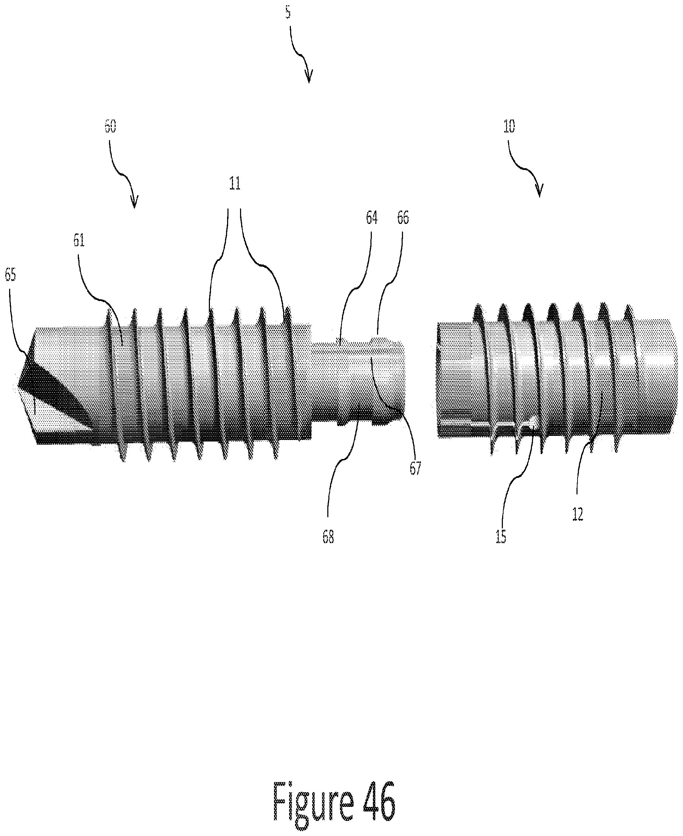

Shown in Figure 45, is a linear apparatus that comprises two parts: an anchor portion 60 and a compressor portion 10. An exploded view is depicted in Figure 46. The compressor portion 10 is substantially the same as the compressor portion in Example 1. The anchor portion 60 in this example is modified to eliminate the need for a coupler portion. Thus, the anchor portion 60 still has an anchor tap edge 65 at one end, but its opposite end is now shaped into a coupling end 68 similar to the coupler portion second end 32, shown in Figure 10.

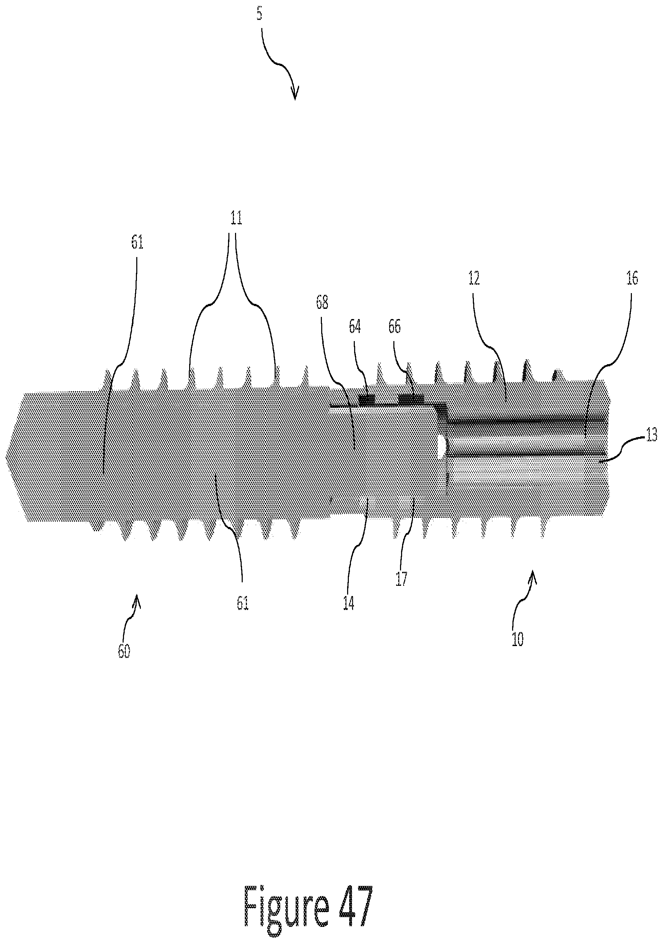

Coupling end 68 is roughly cylindrical in shape and has an external diameter that is sized to fit within compressor portion 10. At least one annular ridge 64 is disposed upon the surface of coupling end 68 for engaging with mating compressor annular groove 14 in compressor 10 (Fig. 47). In a preferred embodiment coupling end 68 has at least two annular ridges 64 and 66 spaced some distance apart longitudinally for engaging in snap-in fashion with at least two annular grooves 14 and 17 of compressor lumen 13. The coupling annular ridges 64 and 66 do not have to be continuous, but can be discontinuous shoulder-type ridges as shown in Figures 46-48.

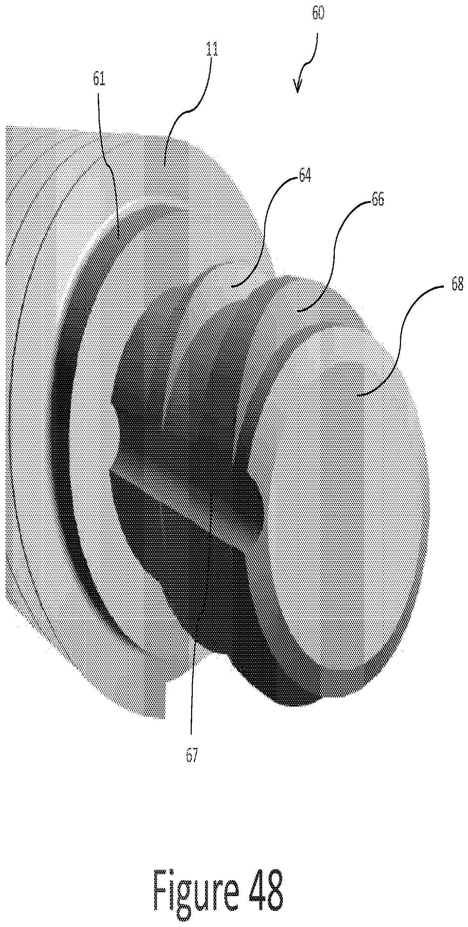

Coupling end 68 also includes an alignment groove 67 that runs longitudinally along its length. Thus, alignment groove 67 runs perpendicular to the annular ridges 64 and 66, thereby disrupting their continuity so that the annular ridges 64 and 66 do not span the entire circumference of coupling end 68 (Figs. 46, 48). Alignment groove 67 is adapted to accommodate an alignment ridge 208 of a delivery tool 200 (Figs. 49-50). In a preferred embodiment, alignment groove 67 is a shallow, roughly hemi-cylindrical depression that runs the entire length of coupling end 68.

Delivery tool 200, shown in Figures 49-52, is similar to delivery tool 100 (Figs. 25-29) but with an altered loading shaft lumen 209 at the end of its loading shaft 204. The loading shaft lumen 209 runs parallel to loading shaft 204. In a preferred embodiment, loading shaft lumen 209 is roughly cylindrical in shape, with a depth and diameter adapted to accommodate coupling end 68 of anchor portion 60. Similarly, loading shaft lumen 209 includes an alignment ridge 208 that runs the entire depth of the lumen and is adapted to fit alignment groove 67. Thus, coupling end 68 of anchor portion 60 will fit into loading shaft lumen 209 only in the orientation in which the alignment ridge 208 and alignment groove 67 are aligned with each other.

Installation of this linear two-part apparatus is similar to the installation procedures described in Examples 2-4. However, because this embodiment does not include a coupler portion, no assembly takes place prior to installation. Rather, the anchor portion and compressor portion are each individually installed, without an accompanying coupler portion, in the manner described in Examples 2 and 3. Delivery tool 200 is used to drive anchor portion 60 into the proximal phalanx, while delivery tool 100 is used to drive compressor portion 10 into the medial phalanx. After both portions are installed, they are coupled by snap-fitting coupling end 68 into compressor lumen 13. Compression of the proximal and medial phalanges can then occur as described in Examples 2-4, using either compressor tool 110 or compressor tool 130, depending on whether compression occurs through the distal phalanx or the medial phalanx.

### Example 6 - Fixed-angle Two-Part PIP Fusion Apparatus

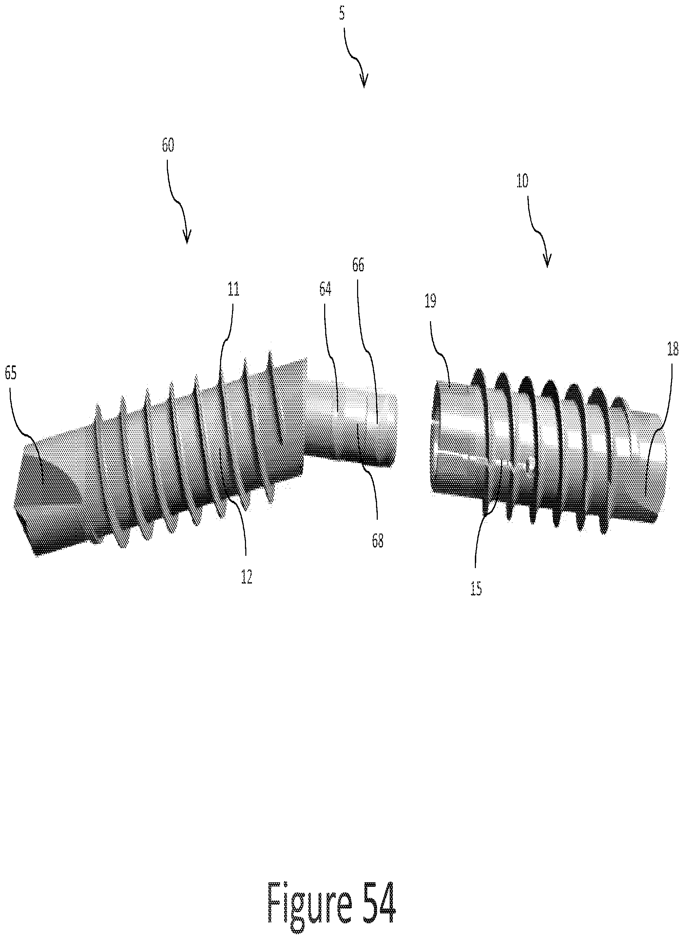

Shown in Figures 53-57, is a two-part apparatus that is similar to the apparatus described in Example 5 but the coupling end 68 of the anchor portion 60 is bent at a fixed angle relative to the anchor axis to provide a more natural conformation of the PIP joint post-surgery. As in the previous example, this embodiment is comprised of both an anchor portion 60 and a compressor portion 10. However, in this embodiment coupling end 68 is bent off-axis from the anchor body 61 of anchor portion 60, as shown in Figures 54-57. The degree of bending will be designed to match the desired degree of natural bending that occurs in either a resting toe or finger PIP joint. In one preferred embodiment, coupling end 68 is bent 15 degrees off-axis from anchor body 61 (Fig. 53). In other preferred embodiments, coupling end 68 is bent 5 or 10 degrees off-axis from anchor body 61. However, numerous degrees of bending are possible to best mimic the natural bend of the PIP joint post-surgery.

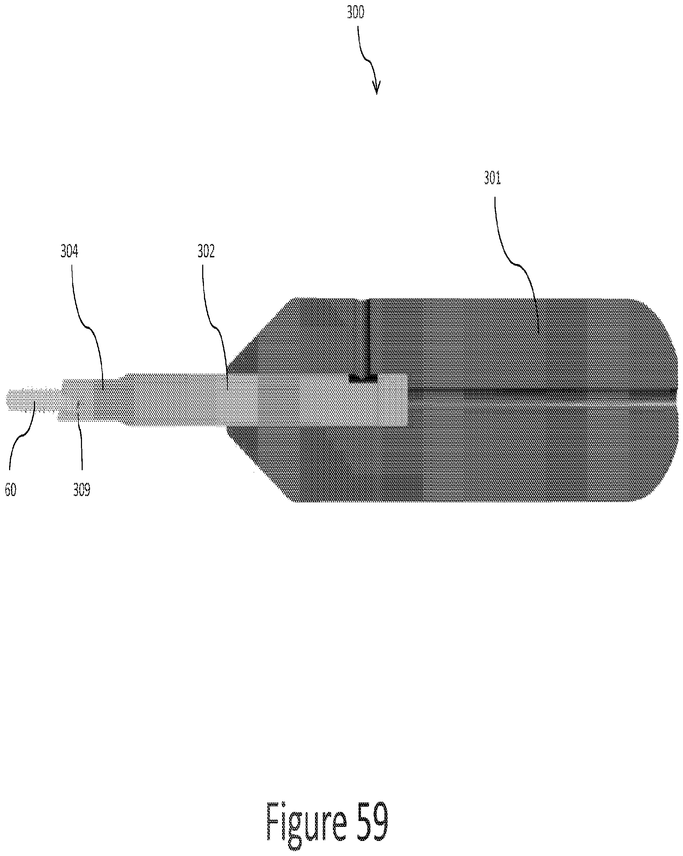

Because of the bent conformation of the anchor portion 60, a slightly modified delivery tool 300, shown in Figures 58-59, must be used to drive the anchor portion 60 into the proximal phalanx. Delivery tool 300 is identical to delivery tool 200 except that the loading shaft lumen 309 is positioned off-axis from the loading shaft 304 (Fig. 59). The degree of off-axis positioning will match that between the coupling end 68 and anchor body 61 of the anchor portion 60. Thus, preferred embodiments will include loading shaft lumens 309 that are positioned 5, 10, and 15 degrees off-axis from the loading shaft 305. As is depicted in Figure 59, this off-axis positioning ensures that the anchor body 61 of anchor portion 60 will not be positioned off-axis when it is driven into the proximal phalanx. In an alternate embodiment of the Delivery Tool 300, the loading shaft lumen portion 309 of the loading shaft 304 may be a separate piece that is rotatably attached to the loading shaft so that the angle of the loading shaft lumen relative to the loading shaft may be adjustable so as to allow the delivery of numerous differently angled fixed-angle anchors, the angle selectable at the option of the physician performing the installation. It will be within the skill of one having ordinary skill in the mechanical arts to design a settable interface between the loading shaft and the separate loading shaft lumen that allows swiveling up to thirty degrees in either direction, while allowing for locking of the angle to accommodate any fixed-angled anchor portion.

The installation procedure of this embodiment is identical to that of Example 5. However, delivery tool 300 must be used in place of delivery tool 200 to drive anchor portion 60 into the proximal phalanx. All other steps of the installation process remain the same.

The embodiments of the invention also comprise kits that include one or more of the bone fusion apparatus of varying sizes and diameters to fit the application, delivery and compression tools, K-wires, drills and drill bits and a case for holding the tools and parts. Components of the kit may be sterile and/or sterilizable (e.g., autoclavable). In some examples, components of the kit, such as bone fusion apparatus and/or wires, may be intended for single use. In some examples, components of the kit, such as drills and/or drivers, may be intended or suitable for repeated use. One embodiment of a kit comprises a flexible bone fusion apparatus comprising:
(a) an anchor portion having an elongated body with screw threads on at least a portion of its exterior, the anchor having a leading tip and an end;
(b) a compressor portion having an elongated body with screw threads on at least a portion of its exterior;
(c) a coupler having a first end adapted to rotatably engage the anchor portion in a first plane, the coupler also having a second end adapted to rotatably engage the compressor through axial rotation;
(d) a delivery tool adapted to hold the flexible bone fusion apparatus during installation, and
(e) a compressor tool for rotating the compressor portion during the compression stage.

It will be understood that various modifications may be made to the embodiments disclosed herein. Therefore, the above description should not be construed as limiting, but merely as exemplifications of preferred embodiments. Those skilled in the art will envision other modifications that come within the scope of the claims appended hereto.

## Claims

1. A flexible bone fusion apparatus (5) comprising:
(a) an anchor portion (60) having an elongated body (61) with screw threads (11) on at least a portion of its exterior, the anchor portion (60) having a leading tip (62) and an end (63);
(b) a compressor portion (10) having an elongated body with screw threads on at least a portion of its exterior; and
(c) a coupler (30) having a first end (22, 31) adapted to rotatably engage the anchor portion (60) in a first plane, the coupler (30) also having a second end (32) adapted to rotatably engage the compressor portion (10) through axial rotation,
wherein the coupler (30) and compressor portion (10) are joined by a snap-fit interface comprising at least one annular ridge (35, 36) in either the compressor portion or coupler but not both, and at least one mating annular groove (14, 17) in the other of either the coupler (30) or compressor portion (10) but not both, said at least one groove (14, 17) being suitable to accommodate said at least one annular ridge (35, 36)
**characterized in that** the diameter at the base of the at least one mating groove is the same as the external diameter of the at least one ridge such that the snap fit interface is adapted to allow relative axial rotation of the compressor portion and coupler.

2. The apparatus of claim 1, **characterized by** one or both of the following features:
(a) wherein the coupler (30) has an anchor end (31) and a compressor end (32), the anchor portion (60) and coupler (30) sharing an interface at which they may rotate in a first plane, the coupler (30) and compressor portion (10) sharing a second interface at which they rotate axially; and
(b) wherein the compressor portion (10) further comprises a lumen (13) running the entire length of the compressor portion (10).

3. The apparatus of claim 1 wherein when assembled the anchor portion (60) and coupler (30) share separate portions of an attachment assembly comprising an interlocking anchor attachment portion (22) and a compressor attachment portion (32).

4. The apparatus of claim 3, **characterized by** one or both of the following features:
(a) wherein the anchor attachment portion (21) comprises at least one locking wedge (23), at least one semi-circular conical face (72) and at least one semi-circular annular groove (25); and
(b) wherein the coupler attachment portion (22) comprises at least one locking wedge (26), at least one semi-circular conical face (42) and at least one semi-circular annular groove (28).

5. The apparatus of claim 2(b) wherein the compressor portion lumen (13) is adapted to engage a delivery tool (100, 200) through a driving pattern, and wherein the compressor portion lumen driving pattern preferably comprises an octagonal cross-section having eight ridges.

6. The apparatus of claim 1, **characterized by** one or both of the following features:
(a) wherein the compressor portion (10) comprises at least one annular groove (14, 17) located in its lumen and the coupler comprises at least one annular ridge (35, 36) located on its external diameter; and
(b) wherein the snap-fit interface has at least one expansion slot cut through either the compressor portion body or coupler body (37) or both.

7. The apparatus of claim 1, **characterized by** one or both of the following features:
(a) wherein the screw threads (11) of the anchor portion (60) and compressor portion (10) are synchronized to provide a continuous thread pitch from anchor portion (60) to compressor portion (10); and
(b) wherein the first plane rotation direction varies from about 0° to about 30° when fixed in final position.

8. A kit comprising:
(a) a flexible bone fusion apparatus (5) comprising
an anchor portion (60) having an elongated body (61) with screw threads (11) on at least a portion of its exterior, the anchor portion (60) having a leading tip (62) and an end (63);
a compressor portion (10) having an elongated body with screw threads on at least a portion of its exterior; and
a coupler (30) having a first end (22, 31) adapted to rotatably engage the anchor portion (10) in a first plane, the coupler (30) also having a second end (32) adapted to rotatably engage the compressor portion (10) through axial rotation,
wherein the coupler (30) and compressor portion (10) are joined by a snap-fit interface comprising at least one annular ridge in either the compressor portion or coupler but not both, and at least one mating annular groove (14, 17) in the other of either the coupler (30) or compressor portion (10) but not both, said at least one groove (14, 17) being suitable to accommodate said at least one annular ridge (35, 36); and
(b) a delivery tool adapted to hold the flexible bone fusion apparatus during installation; and
(c) a compressor tool for rotating the compressor portion during the compression stage
**characterized in that** the diameter at the base of the at least one mating groove is the same as the external diameter of the at least one ridge such that the snap fit interface is adapted to allow relative axial rotation of the compressor portion and coupler.

## Patentansprüche

1. Flexible Knochenverbindungsvorrichtung (5) umfassend:
(a) einen Ankerabschnitt (60) mit einem langgestreckten Körper (61) mit Schraubengewinden (11) an mindestens einer Außenseite, wobei der Ankerabschnitt (60) eine vordere Spitze (62) und ein Ende (63) aufweist;
(b) einen Verdichtungsabschnitt (10) mit einem langgestreckten Körper mit Schraubengewinden an mindestens einer Außenseite; und
(c) ein Verbindungsstück (30) mit einem ersten Ende (22, 31), das so angepasst ist, dass es drehbar in einer ersten Ebene in den Ankerabschnitt (60) eingreifen kann, wobei das Verbindungsstück (30) auch ein zweites Ende (32) aufweist, welches so angepasst ist, dass es durch axiale Drehung drehbar in den Verdichtungsabschnitt (10) eingreifen kann,
wobei das Verbindungsstück (30) und der Verdichtungsabschnitt (10) durch eine Schnittstelle eines Schnappverschlusses verbunden sind, umfassend mindestens einen ringförmigen Steg (35, 36) entweder am Verdichtungsabschnitt oder an dem Verbindungsstück, aber nicht an beiden, und mindestens eine passende Ringnut (14, 17) an dem jeweils anderen, entweder an dem Verbindungsstück (30) oder dem Verdichtungsabschnitts (10), aber nicht an beiden, wobei die mindestens eine Nut (14, 17) geeignet ist, den mindestens einen ringförmigen Steg (35, 36) aufzunehmen,
**dadurch gekennzeichnet, dass** der Durchmesser an der Basis der mindestens einen Passnut gleich dem Außendurchmesser des mindestens einen Stegs ist, so dass die Schnappverschlussschnittstelle so angepasst ist, dass sie eine relative axiale Drehung des Verdichtungsabschnitts und des Verbindungsstücks ermöglicht.

2. Vorrichtung nach Anspruch 1, **gekennzeichnet durch** eines oder beide der folgenden Merkmale:
(a) wobei das Verbindungsstück (30) ein Ankerende (31) und ein Verdichtungsende (32) aufweist, wobei der Ankerabschnitt (60) und das Verbindungsstück (30) sich eine Schnittstelle teilen, um welche sie sich in einer ersten Ebene drehen können, wobei das Verbindungsstück (30) und der Verdichtungsabschnitt (10) sich eine zweite Schnittstelle teilen, an der sie sich in axialer Richtung drehen; und
(b) wobei der Verdichtungsabschnitt (10) ferner einen Lumen (Hohlraum) (13) umfasst, der durch die gesamte Länge des Verdichtungsabschnitts (10) verläuft.

3. Vorrichtung nach Anspruch 1, wobei sich der Ankerabschnitt (60) und das Verbindungsstück (30) im zusammengebauten Zustand, getrennte Abschnitte einer Befestigungsanordnung mit einem verriegelten Ankerbefestigungsabschnitt (22) und einem Verdichtungsbefestigungsabschnitt (32) teilen.

4. Vorrichtung nach Anspruch 3, **gekennzeichnet durch** eines oder beide der folgenden Merkmale:
(a) wobei der Ankerbefestigungsabschnitt (21) mindestens einen Verriegelungskeil (23) aufweist, mindestens eine halbkreisförmige konische Fläche (72) und mindestens eine halbkreisförmige Ringnut (25); und
(b) wobei der Kupplungsbefestigungsabschnitt (22) mindestens einen Verriegelungskeil (26), mindestens eine halbkreisförmige konische Fläche (42) und mindestens eine halbkreisförmige Ringnut (28) aufweist.

5. Vorrichtung nach Anspruch 2 (b), bei dem der Verdichtungsabschnitts-Lumen (13) so ausgebildet ist, dass ein Zustellwerkzeug (100, 200) durch die Profilierung eingreifen kann und wobei die Verdichtungsabschnitts-Lumen-Profilierung vorzugsweise einen achteckigen Querschnitt mit acht Kämmen aufweist.

6. Vorrichtung nach Anspruch 1, **gekennzeichnet durch** eines oder beide der folgenden Merkmale:
(a) wobei der Verdichtungsabschnitt (10) mindestens eine ringförmige Nut (14, 17) aufweist, die in seinem Lumen angeordnet ist, und das Verbindungsstück mindestens einen an dessen Außendurchmesser befindlichen Ringsteg (35, 36) aufweist; und
(b) wobei die Schnappverschlussschnittstelle mindestens einen Erweiterungssteckplatz aufweist, der entweder durch den Verdichtungsabschnittkörper oder den Verbindungsstückkörper (37) oder beide geht.

7. Vorrichtung nach Anspruch 1, **gekennzeichnet durch** eines oder beide der folgenden Merkmale:
(a) wobei die Schraubengewinde (11) des Ankerabschnitts (60) und des Verdichtungsabschnitts (10) synchronisiert sind, um eine kontinuierliche Gewindesteigung von dem Ankerabschnitt (60) zum Verdichtungsabschnitt (10) zu liefern; und
(b) wobei die Drehrichtung der ersten Ebene von etwa 0 ° bis etwa 30 ° variiert, wenn es in endgültiger Position fixiert ist.

8. Ein Bausatz, umfassend:
(a) eine flexible Knochenverbindungsvorrichtung (5), umfassend
einen Ankerabschnitt (60) mit einem langgestreckten Körper (61) mit Schraubengewinden (11) an mindestens einem Außenabschnitt, wobei der Ankerabschnitt (60) eine vordere Spitze (62) und ein Ende (63) aufweist;
einen Verdichtungsabschnitt (10) mit einem langgestreckten Körper mit Schraubengewinden an mindestens einem Außenabschnitt; und
ein Verbindungsstück (30) mit einem ersten Ende (22, 31), das so angepasst ist, dass es drehbar in einer ersten Ebene in den Ankerabschnitt (10) eingreifen kann, wobei das Verbindungsstück (30) auch ein zweites Ende (32) aufweist, das geeignet ist, drehbar in den Verdichtungsabschnitt (10) durch eine axiale Drehung einzugreifen,
wobei das Verbindungsstück (30) und der Verdichtungsabschnitt (10) durch eine Schnittstelle eines Schnappverschlusses verbunden sind mit mindestens einem ringförmigen Steg entweder am Verdichtungsabschnitt oder an dem Verbindungsstück, aber nicht an beiden, und mindestens einer passenden Ringnut (14, 17) an dem jeweils anderen, entweder an dem Verbindungsstück (30) oder an dem Verdichtungsabschnitt (10), aber nicht an beiden, und wobei die mindestens eine Nut (14, 17) geeignet ist, den mindestens einen ringförmigen Steg (35, 36) aufzunehmen; und
(b) ein Zustellwerkzeug, das geeignet ist, die flexible Knochenfusionsvorrichtung während der Montage zu halten; und
(c) ein Verdichtungswerkzeug zum Drehen des Verdichtungsabschnitts während der Verdichtungsphase
**dadurch gekennzeichnet, dass** der Durchmesser an der Basis der mindestens einen Passnut gleich dem Außendurchmesser des mindestens einen Stegs ist, so dass die Schnappverschlussschnittstelle so angepasst ist, dass sie eine relative axiale Drehung des Verdichtungsabschnitts und des Verbindungsstücks ermöglicht.

## Revendications

1. Appareil de fusion osseuse flexible (5) comprenant:
(a) une partie d'ancrage (60) ayant un corps allongé (61) avec des filetages de vis (11) sur au moins une partie de son extérieur, la partie d'ancrage (60) ayant une pointe d'attaque (62) et une extrémité (63) ;
(b) une partie de compresseur (10) ayant un corps allongé avec des filetages de vis sur au moins une partie de son extérieur ; et
(c) un coupleur (30) ayant une première extrémité (22, 31) adaptée pour se mettre en prise, de manière rotative, avec la partie d'ancrage (60) dans un premier plan, le coupleur (30) ayant également une seconde extrémité (32) adaptée pour se mettre en prise, de manière rotative, avec la partie de compresseur (10) par le biais de la rotation axiale,
dans lequel le coupleur (30) et la partie de compresseur (10) sont assemblés par une interface d'encliquetage comprenant au moins une arête annulaire (35, 36) dans la partie de compresseur ou le coupleur, mais pas les deux, et au moins une rainure annulaire de couplage (14, 17) dans l'autre parmi le coupleur (30) ou la partie de compresseur (10), mais pas dans les deux, ladite au moins une rainure (14, 17) étant appropriée pour loger ladite au moins une arête annulaire (35, 36), **caractérisé en ce que** le diamètre, à la base de la au moins une rainure de couplage, est le même que le diamètre externe de la au moins une arête de sorte que l'interface d'encliquetage est adaptée pour permettre la rotation axiale relative de la partie de compresseur et du coupleur.

2. Appareil selon la revendication 1, **caractérisé par** l'une ou les deux des caractéristiques suivantes:
(a) dans lequel le coupleur (30) a une extrémité d'ancrage (31) et une extrémité de compresseur (32), la partie d'ancrage (60) et le coupleur (30) partageant une interface à laquelle ils peuvent tourner dans un premier plan, le coupleur (30) et la partie de compresseur (10) partageant une seconde interface à laquelle ils tournent de manière axiale; et
(b) dans lequel la partie de compresseur (10) comprend en outre une lumière (13) s'étendant sur toute la longueur de la partie de compresseur (10).

3. Appareil selon la revendication 1, dans lequel, lorsqu'ils sont assemblés, la partie d'ancrage (60) et le coupleur (30) partagent des parties séparées d'un ensemble de fixation comprenant une partie de fixation d'ancrage de verrouillage (22) et une partie de fixation de compresseur (32).

4. Appareil selon la revendication 3, **caractérisé par** l'une ou les deux des caractéristiques suivantes:
(a) dans lequel la partie de fixation d'ancrage (21) comprend au moins une cale de verrouillage (23), au moins une face conique semi-circulaire (72) et au moins une rainure annulaire semi-circulaire (25); et
(b) dans lequel la partie de fixation de coupleur (22) comprend au moins une cale de verrouillage (26), au moins une face conique semi-circulaire (42) et au moins une rainure annulaire semi-circulaire (28).

5. Appareil selon la revendication 2(b), dans lequel la lumière de la partie de compresseur (13) est adaptée pour se mettre en prise avec un outil de pose (100, 200) par le biais d'un modèle d'entraînement, et dans lequel le modèle d'entraînement de la lumière de la partie de compresseur comprend de préférence une section transversale octogonale ayant huit arêtes.

6. Appareil selon la revendication 1, **caractérisé par** l'une ou les deux des caractéristiques suivantes:
(a) dans lequel la partie de compresseur (10) comprend au moins une rainure annulaire (14, 17) positionnée dans sa lumière et le coupleur comprend au moins une arête annulaire (35, 36) positionnée sur son diamètre externe; et
(b) dans lequel l'interface d'encliquetage a au moins une fente d'expansion découpée à travers le corps de la partie de compresseur ou le corps de coupleur (37) ou les deux.

7. Appareil selon la revendication 1, **caractérisé par** l'une ou les deux des caractéristiques suivantes:
(a) dans lequel les filetages de vis (11) de la partie d'ancrage (60) et la partie de compresseur (10) sont synchronisés pour fournir un pas de filetage continu à partir de la partie d'ancrage (60) jusqu'à la partie de compresseur (10); et
(b) dans lequel la première direction de rotation de plan varie d'environ 0° à environ 30° lorsqu'elle est fixe dans la position finale.

8. Kit comprenant:
(a) un appareil de fusion osseuse flexible (5) comprenant:
une partie d'ancrage (60) ayant un corps allongé (61) avec des filetages de vis (11) sur au moins une partie de son extérieur, la partie d'ancrage (60) ayant une pointe d'attaque (62) et une extrémité (63);
une partie de compresseur (10) ayant un corps allongé avec des filetages de vis sur au moins une partie de son extérieur; et
un coupleur (30) ayant une première extrémité (22, 31) adaptée pour se mettre en prise, de manière rotative, avec la partie d'ancrage (10) dans un premier plan, le coupleur (30) ayant également une seconde extrémité (32) adaptée pour se mettre en prise, de manière rotative, avec la partie de compresseur (10) par le biais de la rotation axiale,
dans lequel le coupleur (30) et la partie de compresseur (10) sont assemblés par une interface d'encliquetage comprenant au moins une arête annulaire dans la partie de compresseur ou le coupleur mais pas les deux, et au moins une rainure de couplage (14, 17) dans l'autre parmi le coupleur (30) ou la partie de compresseur (10) mais pas les deux, ladite au moins une rainure (14, 17) étant appropriée pour loger ladite au moins une arête annulaire (35, 36); et
(b) un outil de pose adapté pour maintenir l'appareil de fusion osseuse flexible pendant l'installation; et
(c) un outil de compresseur pour faire tourner la partie de compresseur pendant l'étape de compression,
**caractérisé en ce que** le diamètre, à la base de la au moins une rainure de couplage est le même que le diamètre externe de la au moins une arête, de sorte que l'interface d'encliquetage est adaptée pour permettre la rotation axiale relative de la partie de compresseur et du coupleur.
